# EUROPEAN PATENT APPLICATION

(11) **EP 4 821 465 A1**
(43) Date of publication of application: **14.10.2026**
(21) Application number: 24899773.6
(22) Date of filing: 02.12.2024
(51) Int. Cl.: A61F 2/90

(54) **RECESS STENT AND MANUFACTURING METHOD THEREFOR**

(30) Priority: 04.12.2023 CN 202311665203; 04.12.2023 CN 202311653972
(71) Applicant: Lifetech Scientific (Shenzhen) Co., Ltd., Shenzhen, Guangdong 518063 (CN)
(72) Inventor: RUI, Shixuan, Shenzhen, Guangdong 518063 (CN); CHEN, Erchong, Shenzhen, Guangdong 518063 (CN)
(74) Representative: Michalski Hüttermann & Partner mbB
(86) International application number: PCT/CN2024/136090
(87) International publication number: WO 2025/119133

(57) **Abstract**

A concaved stent and a manufacturing method therefor are provided. The concaved stent (100) includes a main stent (10) and a supporting cover (4). The main stent (10) is tubular and has an inner cavity. A side surface of the main stent (10) is recessed toward the inner cavity to form a concave (5). The concave (5) includes a concave bottom (51). The supporting cover (4) is connected to the main stent (10), and at least a portion of the supporting cover (4) and the concave bottom (51) form a radial gap in the radial direction of the concaved stent (100). The supporting cover (4) includes a mesh structure (4a) braided from a braiding wire (40). The mesh structure (4a) includes a plurality of deformable mesh openings. The concave (5) communicates with the outside via the mesh openings. The mesh structure includes a braiding starting end (B0) and a braiding tail end (E0). The braiding starting end (B0) and the braiding tail end (E0) are fixed to each other through a fixing member (8). The concaved stent can mitigate the obstruction to a guide wire or an external branch stent caused by the supporting cover.

## Description

### Technical Field

The present invention relates to the technical field of medical instruments, and in particular, to a concaved stent and a manufacturing method therefor.

### Background Art

Traditional open surgery for vascular diseases such as aortic aneurysms and aortic dissections involves significant trauma, high mortality rates, prolonged operative time, high postoperative complication rates, high surgical difficulty, and the like. In contrast, thanks to the advantages of reduced trauma, high safety, high effectiveness, and the like, minimally invasive interventional surgery is used to treat the vascular diseases. It receives the approval from both doctors and patients and has gradually become a primary manner for treating the vascular diseases. Interventional therapy refers to implanting vascular stents into a diseased vascular segment of a patient by using a delivery system. The implanted vascular stent can support a narrowed, occluded vascular segment or occlude a dissection tear by expansion, to reduce elastic recoil and remodeling of the blood vessel, maintain an unobstructed blood flow in a lumen, and prevent vascular stenosis.

When an aneurysm or an aortic dissection is in a position close to a branch blood vessel on the aorta, a stent with a concave corresponding to the branch blood vessel can be implanted. This ensures that blood in the aorta can enter the branch blood vessel through the concave, thus not only supporting the narrowed, occluded vascular segment or occluding the dissection tear, but also maintaining an unobstructed blood supply to the branch blood vessel. For a blood vessel with a stenotic or tortuous true lumen, a supporting cover can be correspondingly placed over the concave. The supporting cover provides a better supporting effect and can prevent the lumen from pushing the concave and compressing the operating space. However, the placement of the supporting cover may easily obstruct a guide wire or an external branch stent.

### Summary of the Invention

To overcome the drawbacks of the prior art, the present invention provides a concaved stent and a manufacturing method therefor, which can mitigate the obstruction to a guide wire or an external branch stent caused by a supporting cover to an extent.

The present invention provides a concaved stent, including:
a main stent, where the main stent is tubular and has an inner cavity, a side surface of the main stent is recessed toward the inner cavity to form a concave, and the concave includes a concave bottom; and
a supporting cover connected to the main stent, where a radial gap in a radial direction of the concaved stent is formed between at least a portion of the supporting cover and the concave bottom, the supporting cover includes a mesh structure braided from a braiding wire, the mesh structure includes a plurality of deformable mesh openings, and the concave communicates with the outside via the mesh openings; the mesh structure includes a braiding starting end and a braiding tail end, and the braiding starting end and the braiding tail end are fixed to each other through a fixing member.

In one embodiment, the fixing member is located at a radial edge and/or an axial edge of the supporting cover.

In one embodiment, the concave includes an axial edge and a radial edge; an edge corner is formed between the axial edge and the radial edge of the concave; the fixing member includes a first axial end and a second axial end; the first axial end of the fixing member is located at the edge corner of the concave or close to the edge corner of the concave; the second axial end of the fixing member is farther away from the edge corner than the first axial end; and the fixing member is arranged along the radial edge of the concave.

In one embodiment, the supporting cover includes an edge wave angle located at an axial end portion; the edge wave angle includes a vertex; the vertex of the edge wave angle is connected to the edge corner of the concave; the fixing member is arranged on the edge wave angle; and the fixing member is adjacent or close to the vertex of the edge wave angle.

In one embodiment, the edge wave angle is connected to a hooking unit; the hooking unit includes a first hooking member and a second hooking member that are movable relative to each other in an axial direction.

In one embodiment, the concave includes a radial edge; the radial edge of the concave has an inner wall; and the fixing member is fixedly connected to the inner wall.

In one embodiment, the fixing member is in sutured connection to the inner wall through a suture; and the suture forms a plurality of suturing fixing points on an outer surface of the fixing member.

In one embodiment, the fixing member includes a fixing sleeve including a sleeve inner cavity; the braiding starting end and the braiding tail end are fixed in the sleeve inner cavity; a braiding wire section adjacent to the braiding starting end is recorded as a first section; a braiding wire section adjacent to the braiding tail end is recorded as a second section; the first section includes a first inner section located inside the fixing sleeve and a first outer section connected to the first inner section and located outside the fixing sleeve; the second section includes a second inner section located inside the fixing sleeve and a second outer section connected to the second inner section and located outside the fixing sleeve; and the first outer section and/or the second outer section are fixedly connected to the inner wall.

In one embodiment, in a circumferential direction of the concaved stent, the supporting cover includes a first mesh region and at least two second mesh regions respectively connected to two sides of the first mesh region; each second mesh region includes a plurality of first direction supporting wires arranged at intervals and a plurality of second direction supporting wires arranged at intervals; the first direction supporting wires and the second direction supporting wires overlap each other to form a plurality of columns of intersecting units and a plurality of columns of deformable mesh openings; and at least one fixing member is disposed in the second mesh region.

In one embodiment, the concave includes a concave opening; the axial distance between at least one fixing member and the proximal end of the concave opening is less than the axial distance between the fixing member and the distal end of the concave opening; or, the axial distance between at least one fixing member and the proximal end of the concave opening is greater than the axial distance between the fixing member and the distal end of the concave opening.

In one embodiment, when the axial distance between the fixing member and the proximal end of the concave opening is less than the axial distance between the fixing member and the distal end of the concave opening, the ratio of the axial distance between the fixing member and the proximal end of the concave opening to an axial length of the concave opening ranges from 5% to 15%; and when the axial distance between the fixing member and the proximal end of the concave opening is greater than the axial distance between the fixing member and the distal end of the concave opening, the ratio of the axial distance between the fixing member and the distal end of the concave opening to the axial length of the concave opening ranges from 5% to 15%.

In one embodiment, the braiding starting end and the braiding tail end are fixed within the fixing member; the braiding starting end and the braiding tail end are opposite to each other in a longitudinal direction of the braiding wire; and the braiding starting end and the braiding tail end are abutted with each other or spaced apart from each other.

In one embodiment, the concaved stent further includes one or more branch stents connected to the main stent; and the branch stents are disposed within the main stent, or at least part of the branch stents is disposed outside the main stent.

The present invention further provides a manufacturing method for a concaved stent, including:
providing a main stent and a supporting cover; and
connecting the supporting cover to the main stent,
where a manufacturing method for the supporting cover including:
   providing a braiding wire;
   braiding the braiding wire into the mesh structure via a plurality of paths, where the mesh structure includes a braiding starting end and a braiding tail end; and
   fixing the braiding starting end to the braiding tail end of the braiding wire through the fixing member.

In one embodiment, the mesh structure is integrally braided from the braiding wire, and the mesh structure has only one braiding starting end and one braiding tail end.

In one embodiment, the plurality of paths include a plurality of first direction paths and a plurality of second direction paths, and the braiding the braiding wire into a mesh structure via a plurality of paths includes:
step A: braiding and forming a first direction braided unit along the first direction paths; and
step B: braiding and forming a second direction braided unit along the second direction paths, and
repeatedly and alternately performing step A and step B until the mesh structure is formed, where the first direction braided unit and the second direction braided unit are alternately formed and connected to each other, and at least one intersection point is formed between the first direction braided unit and the second direction braided unit that are adjacent to each other.

The present invention further provides a concaved stent, including:
a main stent, where the main stent is tubular and has an inner cavity, a side surface of the main stent is recessed toward the inner cavity to form a concave, and the concave includes a concave bottom; and
a supporting cover connected to the main stent, where a radial gap in a radial direction of the concaved stent is formed between at least a portion of the supporting cover and the concave bottom; the supporting cover enables the concave to communicate with the outside; and a protective structure is arranged on a surface of part or all of the supporting cover.

In one embodiment, the protective structure includes one or more protective layers among a metal layer, a polymer layer, a ceramic layer, and a composite material layer that are arranged on a surface of the supporting cover; and the composite material layer is made of one or more materials among metal, polymer, and ceramic.

In one embodiment, the supporting cover includes a mesh structure braided from a braiding wire; the mesh structure includes a plurality of mesh openings and includes a plurality of overlapping points formed by overlapping of the braiding wire; and at at least some of the overlapping points, a protective structure is arranged on a surface of the braiding wire; and/or, the mesh openings are deformable mesh openings; and the protective structure is arranged on inner walls of at least some of the mesh openings.

In one embodiment, the mesh structure includes a mesh main body and a side connecting member arranged on a radial side edge of the mesh main body; a protective structure is arranged on a surface of the mesh main body; and no protective structure is arranged on at least a portion of the side connecting member.

In one embodiment, the protective structure includes a protective unit; the supporting cover includes at least one column of hooking units; each hooking unit includes a first hook and a second hook that are sequentially hooked to each other in a direction from a proximal end to a distal end; a hooking gap is formed between a valley of the first hook and a peak of the second hook, so that the valley of the first hook and the peak of the second hook are movable relative to each other in the axial direction; and at least one hooking unit is provided with the protective unit.

In one embodiment, the protective structure includes a first protective unit and/or a second protective unit; the first protective unit is arranged on one side facing the hooking gap of the valley of the first hook; and the second protective unit is arranged on one side facing the hooking gap of the peak of the second hook.

In one embodiment, a first protective unit is arranged on one side facing the hooking gap of the valley of the first hook; a second protective unit is arranged on one side facing the hooking gap of the peak of the second hook; the first protective unit and the second protective unit jointly shield part of the hooking gap, and a through hole is formed between the first protective unit and the second protective unit; the valley of the first hook and the peak of the second hook are able to move away from each other in the axial direction, to drive the through hole to be enlarged for insertion of an external branch stent; and in a naturally expanded state, an area of the through hole is smaller than a cross-sectional area of the external branch stent.

In one embodiment, the protective structure includes a protective unit; and the protective unit includes one or more of a sheet-like structure, a strip-shaped structure, and a block-shaped structure.

In one embodiment, at least one of the first protective unit and the second protective unit includes a main sheet and an extension sheet; both the main sheet and the extension sheet are sheet-like structures; one end of the main sheet is connected to the hooking unit, and another end extends into the hooking gap and is connected to the extension sheet; the extension sheet has a free end and extends toward the concave bottom; and the free end of the extension sheet is located between the supporting cover and the concave bottom.

In one embodiment, at least one of the first protective unit and the second protective unit includes a sheet-like structure, and the sheet-like structure has a movable end which includes a thin-walled region; the thickness of the thin-walled region is less than the thickness of another region of the protective unit; and when the external branch stent is inserted into the hooking gap from the outside toward the concave, at least a portion of the thin-walled region deforms relative to another region of the sheet-like structure and extends toward the concave bottom.

In one embodiment, the thin-walled region includes at least one crack-prone strip; the crack-prone strip cracks to form a crack when the external branch stent is inserted into the hooking gap from the outside toward the concave bottom; and at least a portion, adjacent to the crack, of the thin-walled region deforms relative to another region of the sheet-like structure and extends toward the concave bottom.

In one embodiment, the protective structure includes a first protective unit and a second protective unit; the first protective unit and the second protective unit jointly shield the hooking gap; or, the protective structure includes a third protective unit which includes a polymer covering film; the polymer covering film completely covers one or more hooking gaps; the valley of the first hooking unit and/or the peak of the second hooking unit are movable in the axial direction; and an insertion hole is formed in at least a portion of the supporting cover.

Beneficial effects of the present invention are as follows: Compared with the existing technology, the supporting cover of the present invention includes the mesh structure braided from the braiding wire, and the mesh structure includes the plurality of deformable mesh openings. The mesh openings can deform under an external force, so that the guide wire and the external branch stent can pass through the mesh openings, thereby mitigating the obstruction to the guide wire or the external branch stent caused by the supporting cover. In addition, the braiding starting end and the braiding tail end of the mesh structure are fixed to each other through the fixing member. This not only facilitates the stable connection between the braiding starting end and the braiding tail end, but also avoids the risk of mechanical property damage in the vicinity of the braiding starting end and the braiding tail end due to welding.

### Brief Description of the Drawings

FIG. 1 is a schematic diagram of the implanted state of a concaved stent of the present invention;
FIG. 2 is a schematic structural diagram of a concaved stent according to a first embodiment of the present invention;
FIG. 3 is a schematic structural diagram of a concave according to the first embodiment of the present invention;
FIG. 4 is a schematic diagram showing the unfolding of a supporting cover according to an embodiment of the present invention;
FIG. 5 is a schematic diagram showing the unfolding of a supporting cover according to another embodiment of the present invention;
FIG. 6 is a schematic diagram of a protective structure according to an embodiment of the present invention;
FIG. 7 is a schematic diagram of a protective structure according to another embodiment of the present invention;
FIG. 8 is a three-dimensional structural diagram of a supporting cover according to an embodiment of the present invention;
FIG. 9 is a partially enlarged view of position A in FIG. 8;
FIG. 10 is a partially enlarged view of position B in FIG. 8;
FIG. 11 is a partially enlarged view of position C in FIG. 8;
FIG. 12 is a partially enlarged view of position D in FIG. 8;
FIG. 13 is a schematic structural diagram of a protective structure according to another embodiment of the present invention;
FIG. 14 is a schematic structural diagram of a protective structure according to still another embodiment of the present invention;
FIG. 15 is a schematic structural diagram of a hooking unit in an embodiment of the present invention;
FIG. 16 is a schematic three-dimensional diagram of a proximal-end region of a concave according to an embodiment of the present invention;
FIG. 17 is a partially enlarged view of position E in FIG. 16;
FIG. 18 is a schematic structural diagram of a protective unit according to a second embodiment of the present invention;
FIG. 19 is a cut-off diagram of position F-F in FIG. 18;
FIG. 20 is a schematic structural diagram of a protective unit according to a third embodiment of the present invention;
FIG. 21 is a schematic structural diagram of a protective unit according to a fourth embodiment of the present invention;
FIG. 22 is a schematic structural diagram of a protective unit according to another embodiment of the present invention;
FIG. 23 is a schematic diagram of a position of a fixing member in a supporting cover according to a fifth embodiment of the present invention;
FIG. 24 is a schematic diagram of a braiding starting end and a braiding tail end being fixed in the fixing member according to a fifth embodiment of the present invention;
FIG. 25 is a schematic diagram of a position of a fixing member in a supporting cover according to another embodiment of the present invention;
FIG. 26 is a schematic diagram of a position of a fixing member in a supporting cover according to still another embodiment of the present invention;
FIG. 27 is a schematic diagram showing the fitting between a fixing member and a concave according to a fifth embodiment of the present invention;
FIG. 28 is a partially enlarged view of a region in which a fixing member is located according to the fifth embodiment of the present invention;
FIG. 29 is a partially enlarged view of the region in which the fixing member is located in FIG. 28;
FIG. 30 is a schematic diagram of braiding paths of a mesh structure according to the fifth embodiment of the present invention;
FIG. 31 is a schematic structural diagram of a concaved stent according to another embodiment of the present invention;
FIG. 32 is a partial schematic structural diagram of a concaved stent according to an embodiment of the present invention; and
FIG. 33 is a schematic structural diagram of a concaved stent according to an embodiment of the present invention.

### Detailed Description of the Invention

To better understand the concept of the present invention, the implementations of the present invention will be specifically explained below in conjunction with the accompanying drawings. The following specific embodiments are merely some embodiments of the present invention, and are not intended to limit the present invention.

For ease of description, spatial relative relationship terms can be used herein to describe a relationship between an element or feature shown in the figure relative to another element or feature, such as "internal", "external", "inner side", "outer side", "beneath", "below", "on", and "above". This spatial relative relationship term means including different orientations of a device in use or operation, in addition to the orientations depicted in the figures. For example, if a device in a drawing is flipped, an element described as being "below other elements or features" or "under other elements or features" will subsequently be oriented as being "above other elements or features" or "over other elements or features". Therefore, the exemplary term "below" can include two orientations: "above" and "below". The device can be oriented in other ways (rotated 90 degrees or in other directions) and space-correlated descriptors used herein can also be explained correspondingly.

To describe the structure of the present application more clearly, the terms "proximal end" and "distal end" are defined as conventional terms in the field of interventional medical instruments. Specifically, the "distal end" refers to an end from which blood flows out, and the "proximal end" refers to an end into which blood flows. For example, after stent implantation, blood flows from the proximal end of the stent toward the distal end. "Axial direction" represents a longitudinal direction of the stent, and "radial direction" represents a direction perpendicular to the "axial direction".

A "wave ring" (also referred to as a waveform ring) in the present invention is a closed ringlike structure, and a "waveform unit" is an arc-shaped structure. The "wave ring" and the "waveform unit" are made by braiding or cutting a metal elastic material or a polymer material. The metal elastic material includes known materials implanted in medical devices or combinations of various biocompatible materials, such as alloys of two or more single metals such as cobalt, chromium, nickel, titanium, magnesium, and iron, and 316L stainless steel, nickel-titanium-tantalum alloy, nitriding iron, iron-manganese alloy, sulfurized iron, carburized iron, or another biocompatible metal elastic material. The metal elastic material may be a non-degradable or degradable material. The polymer material includes one or a blend of several, or a copolymer of at least two monomers, selected from the following: polylactic acid, polyglycolic acid, polybutylene succinate, poly(β-hydroxybutyrate), polycaprolactone, poly(adipic acid), gylcol ester, a polylactic acid-glycolic acid copolymer, a polyhydroxybutyrate valerate copolymer, polyhydroxyalkanoate, and poly(β-malate). Alternatively, the polymer material may be a biocompatible material such as starch, cellulose, polysaccharides, chitin, chitosan, or derivatives thereof. Both the "wave ring" and the "waveform unit" have radial expansion abilities, and can achieve radial contraction under an external force and self-expand or return to their initial shapes through mechanical expansion (such as balloon dilatation expansion) and maintain the initial shapes after the external force is removed. Therefore, after the stent is implanted into a lumen, they can be clung to an inner wall of the lumen by their radial supporting forces. Waveforms of waves in the "wave ring" and the "waveform unit" are not limited, including Z-shaped waves, M-shaped waves, V-shaped waves, sine waves, and the like. Each of the "wave ring" and "waveform unit" includes a plurality of peaks (also referred to as proximal vertexes), a plurality of valleys (also referred to as distal vertexes), and wave rods for connecting adjacent peaks and valleys. One vertex (a proximal vertex or a distal vertex) and two wave rods connected to the vertex form a wave.

A "covering film" in the present invention can isolate liquid to an extent, and may be made of a polymer material with good biocompatibility such as poly tetra fluoroethylene (PTFE) and polyethylene terephthalate (PET).

### First embodiment

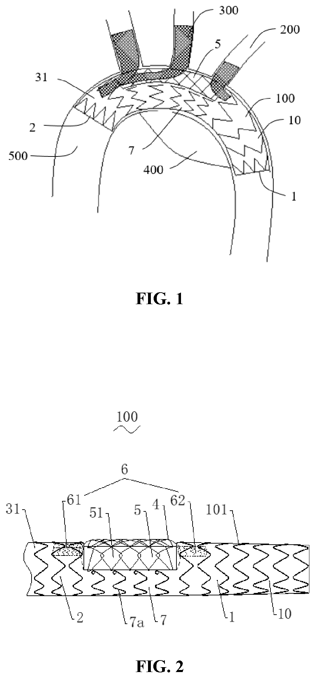

Referring to FIG. 1, a concaved stent 100 of this embodiment is configured to be implanted into a target cavity. The above target cavity may be any cavity within an organism. The present invention does not limit the type of the target cavity. For the convenience of understanding, the present invention uses an aortic arch 500 as the target cavity for illustration. Referring to FIG. 1, the aortic arch 500 is connected to three branch blood vessels 200. The branch blood vessels 200 are connected to a greater curvature side of the aortic arch 500. Blood flows from the aortic arch 500 to the branch blood vessels 200, and an aneurysm 400 (for illustration only; in other cases, the aneurysm 400 may be distributed in other positions of the aortic arch 500) is formed on a lesser curvature side of the aortic arch 500. By implanting the concaved stent 100 into the aortic arch 500 to isolate the aneurysm 400, the blood flowing within the concaved stent 100 cannot in contact with the aneurysm 400, thus ultimately achieving the goal of treating the aneurysm 400. The three branch blood vessels 200 may also be implanted into an external branch stent 300. The external branch stent 300 is connected to the concaved stent 100. The blood inside the concaved stent 100 enters the branch blood vessels 200 through the external branch stent 300.

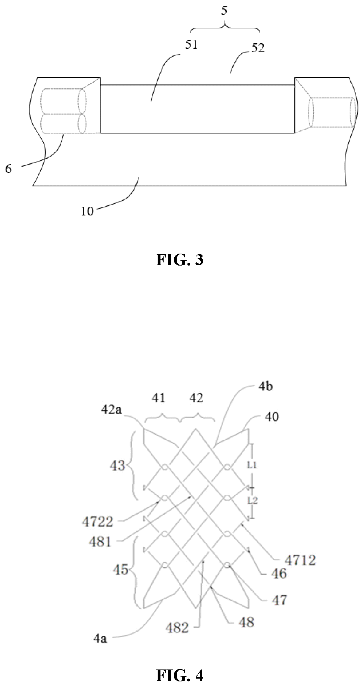

Referring to FIG. 2 and FIG. 3, the concaved stent 100 of this embodiment includes a main stent 10 and a supporting cover 4 connected to the main stent 10.

The entire main stent 10 is of a hollow tubular structure with openings in two ends, and a side surface of the main stent 10 is recessed toward an inner cavity of the main stent to form a concave 5. The concave 5 includes a concave bottom 51 and a concave opening 52. The concave opening 52 and the concave bottom 51 are opposite to each other in a radial direction of the concaved stent 100, and the concave opening 52 faces toward a radial outer side of the concaved stent 100. The supporting cover 4 is connected to the concave 5, and a radial gap is formed in the radial direction of the concaved stent 100 between at least a portion of the supporting cover 4 and the concave bottom 51. The radial gap communicates with the inner cavity of the main stent 10. In this embodiment, a central angle corresponding to a projection of the supporting cover 4 on a radial plane of the concaved stent 100 may be less than or equal to 180 degrees, such as 120 degrees, so as to ensure that the supporting cover 4 has a good radial supporting force and ensures that the inner cavity of the main stent 10 has sufficient space for the blood to flow through. In other embodiments, an intermediate section 7 can be recessed as a whole, and the supporting cover 4 is tubular and surrounds outside the intermediate section 7.

For example, the main stent 10 includes a main supporting portion and a main covering film 31. The main covering film 31 may be arranged on an inner surface and/or an outer surface of the main supporting portion. For example, the main covering film 31 can be arranged only on the outer surface of the main supporting portion. The main covering film 31 may be arranged on part of, or an entirety of, the inner surface of the main supporting portion, or the main covering film 31 may be arranged on part of, or an entirety of, the outer surface.

Referring to FIG. 2, the main stent 10 may be divided in an axial direction into a proximal section 2, a distal section 1, and the intermediate section 7 located between the proximal section 2 and the distal section 1. The proximal section 2 includes a tubular proximal supporting portion and a proximal main covering film. The proximal main covering film may be applied to an inner surface and/or an outer surface of the proximal supporting portion in a manner of suturing, bonding, hot melting, or the like. The proximal supporting portion includes a plurality of main wave rings 101 arranged at intervals in the axial direction. The distal section 1 includes a tubular distal supporting portion and a distal main covering film. The distal supporting portion includes a plurality of main wave rings 101 arranged at intervals in the axial direction. The distal main covering film may be applied to an inner surface and/or an outer surface of the distal supporting portion in a manner of suturing, bonding, hot melting, or the like.

The intermediate section 7 includes an intermediate main covering film and an intermediate supporting portion 7a (which can be omitted in other embodiments). An inner cavity enclosed by the intermediate main covering film communicates with an inner cavity enclosed by the proximal main covering film and an inner cavity enclosed by the distal main covering film. The intermediate supporting portion 7a includes a plurality of arc-shaped waveform units arranged at intervals in the axial direction, and openings of the arc-shaped waveform units face toward the concave 5. A side surface of the main stent 10 located on the intermediate section 7 is recessed toward the inner cavity of the main stent 10 to form the concave 5. A projection of an edge of the concave 5 on a plane passing through two radial side edges of the concave is substantially rectangular (or, the opening of the concave 5 is substantially rectangular). In other embodiments, the concave 5 may be in another shape. Part of the intermediate main covering film serves as a bottom covering film of the concave bottom 51, and the concave bottom 51 may be further provided with a bottom supporting member. The bottom supporting member may include one or more of a waveform unit or a mesh structure. It can be understood that the bottom supporting member can be omitted.

Two sides of the supporting cover 4 in the radial direction are fixedly connected to the intermediate main covering film in a manner of suturing, bonding, hot melting, or the like, and at least a portion of the supporting cover 4 and an outer surface of the concave bottom 51 form a radial gap (or referred to as a space, a voice, or a cavity). An internal branch stent 6 may be disposed within the proximal section 2 and/or the distal section 1. An internal cavity of the internal branch stent 6 is communicated with the internal cavity of the main stent 10, and is also communicated with the radial gap formed between the concave bottom 51 and the supporting cover 4. A plurality of internal branch stents 6 may be provided. For example, the internal branch stents 6 are respectively arranged at a position close to a proximal end of the concave 5 and a position close to a distal end of the concave 5.

Referring to FIG. 2 and FIG. 4, in one embodiment, the supporting cover 4 is of an arc-shaped structure in a circumferential direction of the concaved stent 100. The supporting cover 4 includes a mesh structure 4a braided from a braiding wire 40. The mesh structure 4a includes a mesh main body 4b and a side connecting member 46 arranged on a radial side edge of the mesh main body 4b. The mesh structure 4a is connected to (for example, fixedly connected to) the main stent 10 through the side connecting member 46. The mesh structure 4a includes a plurality of overlapping points (or intersecting points) formed by overlapping (or referred to as intersection) of the braiding wire 40. At the overlapping points, the braiding wires 40 can slide relatively. The mesh structure 4a further includes a plurality of deformable mesh openings communicating with the outside for easy insertion of a guide wire and the external branch stent 300. The supporting cover 4 may be formed by integral braiding of a braiding wire made of shape memory alloy or another material, or by separate braiding and splicing. In other embodiments, the mesh structure 4a of the supporting cover 4 may be formed by cutting. Or, in other embodiments, the supporting cover 4 may not include the mesh structure 4a, but may include a hollow structure with through holes, or another structure with through holes, such as a structure formed by a plurality of waveform rings arranged at intervals in the axial direction.

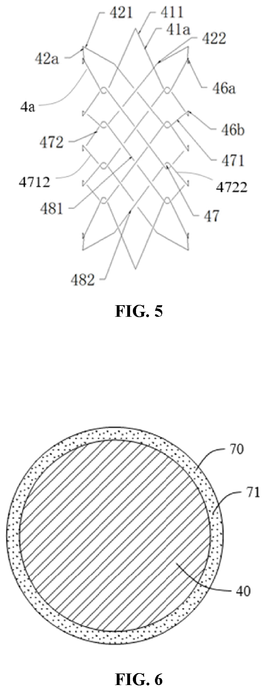

Referring to FIG. 6, a protective structure 70 is arranged on the surface of at least a portion of the supporting cover 4. For example, a protective structure 70 is arranged on the surface of the braiding wire 40 in at least a portion of the supporting cover 4. The protective structure 70 includes one or more protective layers 71 among a metal layer, a polymer layer, a ceramic layer, and a composite material layer. The composite material layer may be made of one or more of metal, polymer, and ceramic. The metal layer may be formed on a surface of the supporting cover 4 in a manner of electroplating, brushing electroplating, physical or chemical deposition, sputtering, spraying, coating, laser deposition, or the like. The metal layer can be made of, but is not limited to, one or more metal materials such as titanium and its alloy, tantalum and its alloy, zirconium and its alloy, niobium and its alloy, zinc alloy, magnesium alloy, iron and its alloys, tungsten and its alloy, platinum and its alloy, gold and its alloy, or the like. The polymer layer may be formed into a film by covering a surface of the supporting cover 4 with a polymer material in a manner of coating, spraying, spinning, fusing, or dipping or a polymer film layer may be wrapped around the surface of the braiding wire 40 in a manner of suturing, bonding, hot melting, or the like, or the polymer layer is arranged on the surface of the braiding wire 40 in any other suitable manner. The polymer layer can be made of one or more of PTFE, PET, expanded polytetrafluoroethylene (ePTFE), fluorinated ethylene propylene (FEP), L-polylactic acid, racemic polylactic acid, polyglycolic acid, a polylactic acid-hydroxyacetic acid copolymer, polyhydroxyalkanoate, polydioxanone, polycaprolactone, polyglucose acid, polyhydroxybutyrate, polyanhydride, polyphosphate ester, polyglycolic acid, and polydioxanone. In other embodiments, the polymer layer may be made of any other suitable material. The ceramic layer may be made of any currently known ceramic material that can be applied to a human body. The ceramic layer may be arranged on the surface of the supporting cover 4 by electroplating, coating, spraying, fusing, or in any other suitable manner. Arranging the protective structure 70 on the surface of the braiding wire 40 in at least a portion of the supporting cover 4 is beneficial for reducing a probability of mechanical performance damage caused by wear on the braiding wire 40 in at least the portion of the supporting cover 4.

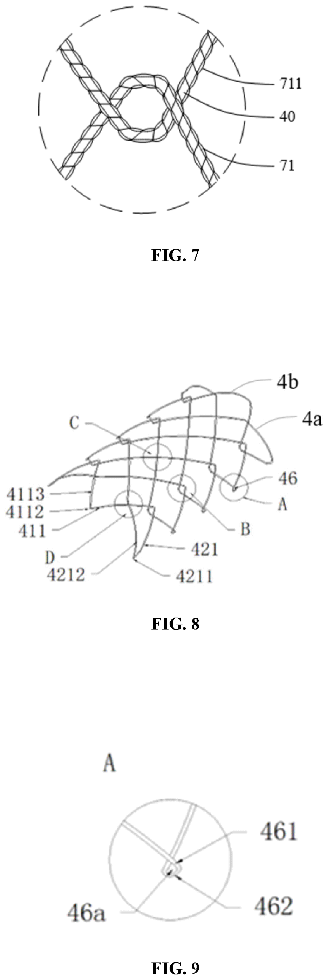

In this embodiment, a protective structure 70 is arranged on an entire surface of the braiding wire 40 of the mesh main body 4b. For example, referring to FIG. 7, during manufacturing, the surface of the braiding wire 40 in the mesh main body 4b is wrapped with a strip 711 (e.g. PTFE strip). For example, the strip 711 can be helically wrapped around the surface of the braiding wire 40 to completely wrap around the surface of the braiding wire 40 in the mesh main body 4b. Next, the supporting cover 4 wrapped with the strip 711 is subjected to thermal treatment to thermally heat the strip 711 onto the surface of the braiding wire 40. In other embodiments, a bioceramic layer serving as the protective layer 70 may be electroplated on the surface of the braiding wire 40 in the mesh main body 4b. In other embodiments, a protective structure 70 made of another material can be selected, and the protective layer 71 can be arranged in any other suitable manner.

In this way, the protective structure 70 is arranged at the overlapping points between all braiding wires 40 in the mesh main body 4b and on the inner walls of all the mesh openings. This can lower the risk of damage to the braiding wire 40 at the overlapping points of the braiding wires 40 that are prone to friction, and is also beneficial for protecting the mesh openings and the external branch stent 300 inserted into the mesh openings. This can lower the risk of damage to the mesh openings or the external branch stent 300 caused by the mutual friction between the mesh openings and the external branch stent 300 inserted into the mesh openings. In other embodiments, the mesh main body 4b may only be partially provided with a protective structure 70. For example, at some or all of the overlapping points of the braiding wires 40, a protective structure 70 is arranged on the surface of the braiding wire 40, and no protective structure 70 is arranged on the inner walls of the mesh openings. Or, a protective structure 70 is arranged on the inner walls of only part or all of the mesh openings, and no protective structure 70 is arranged at the overlapping points of the braiding wire 40. Or, only at some of the overlapping points of the braiding wires 40, a protective structure 70 is arranged on the surface of the braiding wire 40, and a protective structure 70 is arranged on the inner walls of part of the mesh openings.

In this embodiment, no protective structure 70 is arranged in part or all of the side connecting member 46 of the supporting cover 4. For example, the side connecting member 46 is fixedly connected to the main stent 10, for example, connected to the main covering film 31 of the main stent 10 in a suturing manner. Therefore, the relative position between the side connecting member 46 and the main stent 10 is relatively fixed. Because of less relative movement, the probability of relative friction is low. Therefore, no protective structure 70 is arranged on at least a portion of the side connecting member 46. In addition, if a protective structure 70 with surface smoothness greater than surface smoothness of a bare side connecting member 46 is arranged on a surface of the side connecting member 46, and the side connecting member 46 is in sutured connection to the main stent 10, relative sliding between the side connecting member 46 and the main stent 10 easily occurs, thereby causing relative displacement between the supporting cover 4 and the main stent 10. Therefore, not arranging a protective structure 70 on the side connecting member 46 is also beneficial for reducing the probability of relative displacement between the supporting cover 4 and the main stent 10. Further, a step exists between a bare region on the side connecting member 46 and a region that is adjacent to the bare region and is provided with a protective structure 70 on the surface, which can provide a limiting effect on a suture for suturing the side connecting member 46 to the main stent 10, thereby further reducing the probability of relative displacement between the supporting cover 4 and the main stent 10.

For example, referring to FIG. 8 and FIG. 9, in this embodiment, the side connecting member 46 is substantially triangular, including two waists 461, a bottom edge 462 connected to the two waists 461, and a connecting hole 46a enclosed by the waists 461 and the bottom edge 462. The connecting hole 46a can serve as a suturing hole for being fixedly connected to the covering film or the main stent 10 via suturing. The connecting hole 46a not only facilitates the suturing operation, but also plays a limiting role, to reduce the relative sliding between a suture and the supporting cover 4. In this embodiment, the connecting hole 46a is a closed (or closed-loop) hole, which is conducive to further enhancing the limiting effect. In other embodiments, the connecting hole 46a may be an open hole. In other embodiments, the connecting hole 46a may not serve as a suturing hole. The side connecting member 46 can also be connected to the main stent 10 in a manner of bonding, hot melting, or the like.

The bottom edge 462 of the side connecting member 46 extends substantially in the axial direction and is connected to a radial edge of the concave 5. A vertex is formed at an intersection between the two waists 461 of the side connecting member 46. At the vertex position, the two waists 461 overlap each other. The bottom edge 462 may be connected to the edge of the concave 5 via suturing or bonding, and overlapping positions of the two waists 461 can slide relative to each other, which is conducive to further increasing the expansion size of a mesh opening on a side edge of the supporting cover 4. A protective structure 70 may be arranged at the overlapping positions of the two waists 461 to lower the risk of damage caused by mutual friction at the overlapping positions of the two waists 461. In other embodiments, the overlapping positions of the two waists 461 may be fixed to each other, for example, are sutured and fixed by a suture. This connection manner can enable the two radial sides of the supporting cover 4 to have better stability, thereby ensuring the overall supporting force of the supporting cover 4 after connection, and lowering the risk of damage caused by mutual friction at the overlapping positions of the two waists 461. In another embodiment, protective structures 70 are arranged on surfaces of the two waists 461, and no protective structure 70 is arranged on the bottom edge 462. The bottom edge 462 is in a sutured connection to the edge of the concave 5, and the two waists 461 with the protective structures 70 can better restrict the suture on the bottom edge 462, to better reduce the probability of significant relative displacement between the side connecting members 46 and the main stent 10. In other embodiments, the side connecting member 46 may be in another shape. For example, the side connecting member 46 may also be in any other suitable shape such as a water droplet shape, a circle, an ellipse, or the like.

There may be various structure of the supporting cover 4 in the present invention. A person skilled in the art can select a structure according to an actual application scenario. The structure of the supporting cover will be illustrated below.

Referring to FIG. 4, the supporting cover 4 includes a first mesh region 41. The first mesh region 41 includes at least one column of hooking units 47. Each column of hooking units 47 includes a plurality of hooking units 47 sequentially arranged in the axial direction. Each hooking unit 47 includes a first hook 471 and a second hook 472. The first hook 471 and the second hook 472 are formed by bending a braiding wire 40. The first hook 471 includes a wave that rises toward a distal end, i.e. includes a valley 4711 (also referred to as a distal vertex) and two first wave rods 4712 connected to the valley 4711. The second hook 472 includes a wave that rises toward a proximal end, i.e. includes a peak 4721 (also referred to as a proximal vertex) and two second wave rods 4722 connected to the peak 4721. In this embodiment, the first hook 471 and the second hook 472 of each hooking unit 47 are hooked to each other substantially in the axial direction. It should be noted that "substantially in the axial direction" here refers to a connecting line between the distal vertex of the first hook 471 and the proximal vertex of the second hook 472 being substantially parallel to an axis of the supporting cover 4, or the angle between the connecting line and the axis of the supporting cover 4 is less than or equal to 45°.

In a naturally expanded state, the first hook 471 and the second hook 472 are hooked to each other, and a hooking gap G (i.e., a distance) exists between the valley 4711 of the first hook 471 and the peak 4721 of the second hook 472. Therefore, the first hook 471 can move toward the proximal end or the distal end, and the second hook 472 may also move toward the proximal end or the distal end. However, the hooking gap G limits the distance that the first hook 471 moves toward the proximal end and the second hook 472 moves toward the distal end. It can be understood that the hooking gap G can serve as a mesh opening of the mesh structure 4a, which can expand under an external force to allow the guide wire or the external branch stent 300 to be inserted into the mesh opening.

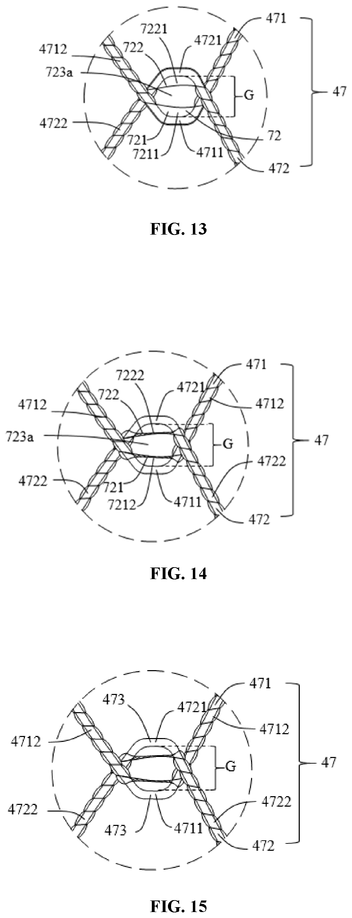

Referring to FIG. 4 and FIG. 13, in this embodiment, the protective structure 70 further includes a protective unit 72, and at least one hooking unit 47 is provided with the protective unit 72. The protective unit 72 may be fixedly connected to the hooking unit 47 via suturing, bonding, hot melting, or the like. Since the area of the hooking gap G is smaller than the area of another mesh opening (another type of mesh opening besides the hooking gap G) of the mesh structure 4a, if the guide wire passes through the hooking gap G and guides the external branch stent 300 to be inserted into the hooking gap, the external branch stent 300 inserted therein can be subjected to a higher compression force applied by the hooking gap G, and the frictional force between them is higher. The arrangement of the protective unit 72 on the inner wall of the hooking gap G is beneficial for reducing the probability of damage to the external branch stent 300 due to the compression and the friction. It can be understood that in other embodiments, only the protective unit 72 may be provided, and no protective layer 71 is provided, or only the protective layer 71 may be provided, and no protective unit 72 is provided.

For example, the protective structure 70 further includes a first protective unit 721 and a second protective unit 722. The first protective unit 721 is arranged on one side facing the hooking gap G of the valley 4711 of the first hook 471, and the second protective unit 722 is arranged on one side facing the hooking gap G of the peak 4721 of the second hook 472. The first protective unit 721 and the second protective unit 722 can jointly shield at least part of the hooking gap G, thereby reducing the probability that the guide wire passes through the hooking gap G and guides the external branch stent 300 to be inserted into the hooking gap G. Even if the external branch stent 300 is inserted into the hooking gap G and connected to a corresponding internal branch stent 6, in a subsequent use process, the first protective unit 721 and the second protective unit 722 can also effectively protect the hooking unit 47 and the external branch stent 300, and can accelerate the endothelialization of the supporting cover 4, thus lowering the risk of damage to the external branch stent 300 caused by a frictional force and a cutting force therebetween. When the first protective unit 721 and the second protective unit 722 only jointly shield part of the hooking gap G, a through hole 723a may be formed between the first protective unit 721 and the second protective unit 722. The valley 4711 of the first hook 471 and the peak 4721 of the second hook 472 can move away from each other in the axial direction, to drive the through hole to be enlarged for insertion of the external branch stent 300. In a naturally expanded state, the area of the through hole 723a is smaller than a cross-sectional area of the external branch stent 300. In other embodiments, the first protective unit 721 and the second protective unit 722 can completely shield the hooking gap G. For example, adjacent edges of the first protective unit 721 and the second protective unit 722 abut against each other, or an overlapping region is provided between the first protective unit 721 and the second protective unit 722, to jointly completely shield the hooking gap G to prevent the guide wire and the external branch stent 300 from being inserted into the hooking gap G.

The protective unit 72 such as the first protective unit 721 and the second protective unit 722 includes one or more of a sheet-like structure, a strip-shaped structure, and a block-shaped structure. Referring to FIG. 13, in this embodiment, each of the first protective unit 721 and the second protective unit 722 includes a sheet-like structure. The sheet-like structure may be made of one or more polymer materials: PTFE, PET, ePTFE, FEP, L-polylactic acid, racemic polylactic acid, polyglycolic acid, a polylactic acid-hydroxyacetic acid copolymer, polyhydroxyalkanoate, polydioxanone, polycaprolactone, polyglucose acid, polyhydroxybutyrate, polyanhydride, polyphosphate ester, polyglycolic acid, and polydioxanone. For example, the first protective unit 721 includes a first sheet-like structure 7211, and the second protective unit 722 includes a second sheet-like structure 7221. The first sheet-like structure 7211 and the second sheet-like structure 7221 jointly shield at least part of the hooking gap G. One end of the first sheet-like structure 7211 is fixedly connected to the valley 4711 of the first hook 471 and regions close to the valley 4711 of the two first wave rods 4712, while another end extends toward the second hook 472. One end of the second sheet-like structure 7221 is fixedly connected to the peak 4721 of the second hook 472 and regions close to the peak 4721 of the two second wave rods 4722, while another end extends toward the first hook 471. The sheet-like structure is beneficial for better shielding the hooking gap G, to reduce the probability of insertion of the external branch stent 300 into the hooking gap G. In addition, the sheet-like structure can prevent, to an extent, excessive deformation because the external branch stent 300 inserted into the hooking gap G is compressed into a narrow space close to the valley 4711 of the first hook 471 and the peak 4721 of the second hook 472. Moreover, the sheet-like structure can provide good buffering, which can effectively lower the risk of damage to the external branch stent 300 caused by the frictional force and the cutting force between the external branch stent 300 and the hooking unit 47. When the sheet-like structure is made of a degradable material, after the concaved stent and the external branch stent 300 subsequently undergo endothelialization, the sheet-like structure can be gradually degraded and absorbed by an organism, thus providing a large accommodating space for the external branch stent 300 inserted.

In this embodiment, the through hole 723a is formed between an end of the first sheet-like structure 7211 farther away from the valley 4711 and an end of the second sheet-like structure 7221 farther away from the peak 4721. The edge of the end of the first sheet-like structure 7211 farther away from the valley 4711 is substantially an arc that bends toward the valley 4711, and the edge of the end of the second sheet-like structure 7221 farther away from the peak 4721 is substantially an arc that bends toward the peak 4721, so that the through hole 723a between the first sheet-like structure 7211 and the second sheet-like structure 7221 is substantially olive-shaped. The size of the olive-shaped through hole 723a in the radial direction (or referred to as a transverse direction) of the supporting cover 4 is larger than the size of the olive-shaped through hole 723a in the axial direction of the supporting cover 4. Even if the external branch stent 300 is inserted into the through hole, the external branch stent 300 can be limited in a region with a larger radial size to avoid the excessive deformation because the external branch stent 300 is compressed into the narrow space close to the valley 4711 of the first hook 471 and the peak 4721 of the second hook 472. In addition, the arc-shaped edges of the first sheet-like structure 7211 and the second sheet-like structure 7221 can provide guidance for the relative movement between the first hook 471 and the second hook 472, thus avoiding damage caused by upwarping of the peak 4721 to an inner wall of a tissue because the concaved stent 100 is raised toward the concave 5 due to deviation of hooking positions of the first hook 471 and the second hook 472.

In this embodiment, the maximum radial size and the maximum axial size of the through hole 723a are both greater than the diameter of the guide wire. The maximum radial size refers to the maximum length of a connecting line between two radial end points of the through hole 723a in the radial direction (or referred to as the transverse direction) of the concave 5, and the maximum axial size refers to the maximum length of a connecting line between two axial end points of the through hole 723a in the axial direction of the concave 5. The guide wire referred to herein is a guide wire that guides the external branch stent 300 to enter the internal branch stent 6. The advantage of the maximum radial size and the maximum axial size of the through hole 723a being both greater than the diameter of the guide wire is that a relative movement space is provided for the first hook 471 and the second hook 472 to enable the supporting cover 4 to have good bending performance. In other embodiments, at least one of the maximum radial size and the maximum axial size of the through hole 723a is less than the diameter of the guide wire. In this way, it is conducive to preventing the guide wire from being inserted into the through hole 723a, thereby reducing the probability of insertion of the external branch stent 300 into the through hole. In other embodiments, the through hole 723a may not be formed between the first sheet-like structure 7211 and the second sheet-like structure 7221, but they jointly completely shield the hooking gap G.

Referring to FIG. 14, in other embodiments, each of the first protective unit 721 and the second protective unit 722 includes a strip-shaped structure. For example, the first protective unit 721 includes a first strip-shaped structure 7212, and the second protective unit 722 includes a second strip-shaped structure 7222. The first strip-shaped structure 7212 and the second strip-shaped structure 7222 jointly shield at least part of the hooking gap G, and the hooking gap G is partitioned into a plurality of through holes 723a. Two ends of the first strip-shaped structure 7212 in the longitudinal direction are respectively connected to the two first wave rods 4712 of the first hook 471, and two ends of the second strip-shaped structure 7222 in the longitudinal direction are respectively connected to the two second wave rods 4722 of the second hook 472. The first strip-shaped structure 7212 and the second strip-shaped structure 7222 may extend substantially in parallel or not in parallel. The strip-like structure is beneficial for reducing the probability of insertion of the external branch stent 300 into the hooking gap G. In addition, the strip-shaped structure can prevent, to an extent, excessive deformation because the external branch stent 300 inserted into the hooking gap G is compressed into a narrow space close to the valley 4711 of the first hook 471 and the peak 4721 of the second hook 472 and can provide good buffering. Therefore, this can effectively lower the risk of damage to the external branch stent 300 caused by the frictional force and the cutting force between the external branch stent 300 and the hooking unit 47. In addition, the strip-shaped structure minimizes limiting the relative movement between the first hook 471 and the second hook 472, and the first hook 471 and the second hook 472 can flexibly move relative to each other, thus better maintaining the bending performance of the supporting cover 4. Meanwhile, the strip-shaped structure is also easily radially folded, so that the supporting cover 4 is more easily radially compressed and may not have a large size after the radial compression. When the strip-shaped structure is made of a degradable material, after the strip-shaped structure is degraded and broken, part of a remaining structure extends into the radial gap between the supporting cover 4 and the concave 5, which is conducive to accelerating the endothelialization in the region of the supporting cover 4. After the endothelialization, the supporting cover 4 and the external branch stent 300 can form an integrated structure, thus further lowering the risk of damage to the external branch stent 300 caused by the frictional force and the cutting force between the external branch stent 300 and the hooking unit 47.

It can be understood that in other embodiments, the first protective unit 721 and the second protective unit 722 may respectively employ different structures. For example, the first protective unit 721 includes a sheet-like structure, while the second protective unit 722 includes a strip-shaped structure, and vice versa. When the first protective unit 721 and the second protective unit 722 may respectively employ different structures, they can have the advantages of various structures. In other embodiments, one protective unit 72 may be provided in the hooking gap G. For example, one of the first protective unit 721 and the second protective unit 722 is omitted. In other embodiments, more than two protective units 72 may be provided in the hooking gap G.

Further, referring to FIG. 15, each of the valley 4711 of the first hook 471 and the peak 4721 of the second hook 472 in this embodiment includes a transverse rod 473. The two first wave rods 4712 of the first hook 471 are connected to each other through the transverse rod 473, while the two second wave rods 4722 of the second hook 472 are connected to each other through the transverse rod 473. The transverse rod 473 is conducive to further enlarging the size of the mesh opening formed by the hooking gap G, so that the hooking gap G is more suitable for serving as a mesh opening for the entry of the external branch stent 300. In addition, in the absence of a transverse rod 473, two wave rods are directly connected to each other, and the wave angle of the peak 4721 or valley 4711 formed by the connection is relatively sharp. When the supporting cover 4 bends with the tubular stent, the wave angle can upwarp on the curved surface. Under long-term use, the wave angle will scratch an inner wall of a blood vessel, causing unnecessary damage. Therefore, the transverse rod 473 can lower the risk of scratching the inner wall of the blood vessel.

The angle formed by the transverse rod 473 and the first wave rod 4712 and/or the second wave rod 4722 is obtuse, to further enlarge the size of the mesh opening formed by the hooking gap G. The length of the transverse rod 473 should be appropriate, ranging from 1 millimeter to 4 millimeters. In this way, effects of enlarging the area of the hooking gap G and protecting the inner wall of the blood vessel can be achieved, and a large radial compression size of the supporting cover 4 caused by an excessively long transverse rod 473 can be avoided. In this embodiment, the transverse rod 473 adopts a straight rod. The straight rod can directly and effectively prevent a vascular wall from being scratched. When the straight rod is used, arc transitioning is used when the straight rod is connected to the two wave rods. In other embodiments, the transverse rod 473 can also adopt an arc-shaped rod. The arc-shaped rod has a smaller degree of curvature, and a preferred curvature range may be from 60° to 140°.

Referring to FIG. 4, in this embodiment, the supporting cover 4 further includes a second mesh region 42 connected to the first mesh region 41. Both the first mesh region 41 and the second mesh region 42 can achieve radial contraction under an external force, and after the external force is removed, they can self-expand to or return to their initial shapes through mechanical expansion (such as balloon dilatation expansion) and maintain their initial shapes. Preferably, in the circumferential direction of the concaved stent 100, the supporting cover 4 includes a second mesh region 42 and two first mesh regions 41 connected to two radial sides of the second mesh region 42.

The second mesh region 42 includes a plurality of first direction supporting wires 481 arranged at intervals and a plurality of second direction supporting wires 482 arranged at intervals. Both the first direction supporting wires 481 and the second direction supporting wires 482 are braiding wires 40. The first direction supporting wires 481 extend substantially in a first direction, and the second direction supporting wires 482 extend substantially in a second direction. The first direction supporting wires 481 and the second direction supporting wires 482 overlap (or referred to as being interwoven with) each other to form a plurality of columns of mesh openings and a plurality of columns of intersecting units 48. Each column of mesh openings includes a plurality of mesh openings arranged substantially in the axial direction. Each column of intersecting units 48 includes a plurality of intersecting units 48 arranged at intervals substantially in the axial direction. Each mesh opening is substantially diamond-shaped, or may be square, rectangular, or in another shape. Four intersecting units 48 are correspondingly arranged at four corners of the mesh opening. Each intersecting unit 48 includes an intersection point formed by the mutual overlapping of each first direction supporting wire 481 and each second direction supporting wire 482. At the intersection point, the first direction supporting wire 481 and the second direction supporting wire 482 can move relative to each other. In some intersecting units 48, the first direction supporting wires 481 are located on outer sides of the second direction supporting wires 482. In some intersecting units 48, the first direction supporting wires 481 are located on inner sides of the second direction supporting wires 482. In other embodiments, the first direction supporting wires 481 in the second mesh region 42 are all located on the outer sides of the second direction supporting wires 482, or the first direction supporting wires 481 are all located on the inner sides of the second direction supporting wires 482. Since the first direction supporting wires 481 and the second direction supporting wires 482 at the intersection points of the intersecting units 48 intersect and are relatively movable, the mesh openings in the second mesh region 42 can deform and enlarge under an external force, to facilitate the guide wire and the external branch stent 300 to pass through the mesh openings. When the external force is removed, the mesh openings can contract to play a role of supporting and limiting the external branch stent 300, thus reducing occurrence of swinging of the external branch stent 300 with blood or heart beats, and ensuring the stability of branch blood supply.

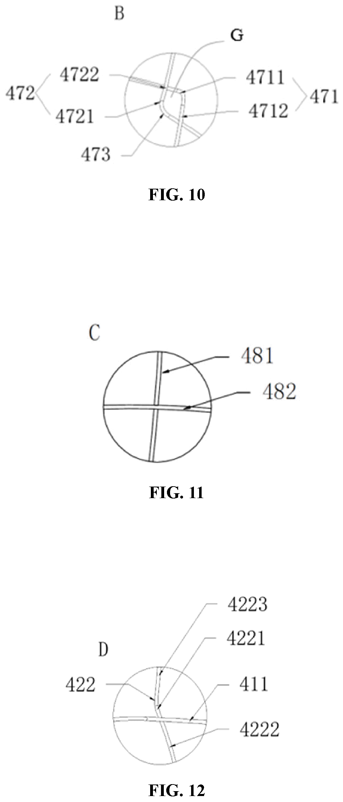

Referring to FIG. 4, the axial direction of the supporting cover 4 from the proximal end to the distal end includes a proximal cover 43 and a distal cover 45. Referring to FIG. 8 to FIG. 12 at the same time, when the first hook 471 and the second hook 472 are hooked to each other to form the hooking unit 47, the hooking unit 47 in the proximal cover 43 is different from the hooking unit 47 in the distal cover 45. In the proximal cover 43, the first wave rod 4712 of the same hooking unit 47 close to a radial edge of the supporting cover 4 spans over a top of the second wave rod 4722; and/or, in the distal cover 45, the second wave rod 4722 of the same hooking unit 47 close to the radial edge of the supporting cover 4 spans over a top of the first wave rod 4712. The top refers to one side farther away from the inner cavity of the main stent 10, which can also be understood as an outer side of an arched structure of the supporting cover 4. The reason for this setting is that if the external branch stent 300 enters the concave 5 from the mesh opening on the proximal cover 43 and is then connected to the internal branch stent 6 located at the proximal section 2, in the proximal cover 43, the first wave rod 4712 of the same hooking unit 47 close to the radial edge of the supporting cover 4 spans over the top of the second wave rod 4722, so that the first wave rod 4712 can be separated from the second wave rod 4722 below it in the radial direction of the concaved stent 100, to have a better upward deformation ability and space. In this way, the mesh opening on a radial side edge of the proximal cover 43 can undergo significant deformation under an external force, to expand large enough to facilitate the guide wire and the external branch stent 300 to pass through the mesh opening. After the external force is removed, the mesh opening can contract to play a role of supporting and limiting the external branch stent 300, thus reducing occurrence of swinging of the external branch stent 300 with blood or heart beats, and ensuring the stability of branch blood supply. On the contrary, if the second wave rod 4722 at the position spans over the top of the first wave rod 4712, the first wave rod 4712 is restricted by the second wave rod 4722 above it, making it difficult for the first wave rod to be further raised. This will cause the first hook 471 and the second hook 472 to be twisted together at a hooking position and is not conducive to the expansion deformation of the mesh opening, thus affecting the entry of the external branch stent 300.

In the distal cover 45, the second wave rod 4722 of the same hooking unit 47 close to the radial edge of the supporting cover 4 spans over the top of the first wave rod 4712. The principle and effect applied by this structure are similar to those applied by the proximal cover 43, and will not be elaborated here.

It can be understood that to achieve easy expansion and deformation of the mesh opening on the radial side edge of the supporting cover 4, it is not required that the structure of the second mesh region 42 is the same as the structure described in this embodiment. In other embodiments, the structure of the second mesh region 42 may be similar to the structure of the first mesh region 41 of this embodiment, including at least one column of hooking units 47, or the structure of the second mesh region 42 may be any other suitable structure.

Referring to FIG. 4, further, in one embodiment, the first wave rod 4712 and the second wave rod 4722 on the radial edge of the supporting cover 4 close to the hooking unit 47 form an axial gap. Maximum axial lengths of the axial gaps formed by the same column of hooking units 47 may be the same or different. For example, in this embodiment, the maximum axial lengths of the axial gaps formed by the same column of hooking units 47 are not the same. For example, a plurality of axial gaps formed between a plurality of hooking units 47 include end gaps and intermediate gaps. The end gaps are closer to axial end portions of the supporting cover 4 than the intermediate gaps. The maximum axial length (L1 shown in FIG. 4) of at least one end gap is greater than the maximum axial length (L2 shown in FIG. 4) of each intermediate gap. For example, the maximum axial length of an end gap close to the proximal end of the supporting cover 4 is greater than the maximum axial length of each intermediate gap, and the maximum axial length of an end gap close to the distal end of the supporting cover 4 is greater than the maximum axial length of each intermediate gap. In other embodiments, if an internal branch stent 6 is arranged at only one axial end portion of the supporting cover 4, the maximum axial length of the end gap close to this axial end portion is greater than the maximum axial length of each intermediate gap. The axial end portion of the supporting cover 4 is opposite to an edge of the internal branch stent 6, so that it is highly possible that the external branch stent 300 enters the internal branch stent 6 from the mesh opening close to the axial end portion of the supporting cover 4. By setting the maximum axial length L1 of the end gap to be greater than the maximum axial length L2 of the intermediate gap, the size of the mesh opening on the side edge close to the axial end portion of the supporting cover 4 of the supporting cover 4 is further increased to facilitate the entry of the guide wire and the external branch stent 300. It can be understood that to achieve easy expansion and deformation of the mesh openings on the radial side edge of the supporting cover 4, it is not necessarily required that the maximum axial length L1 of the end gap is greater than the maximum axial length L2 of the intermediate gap. The maximum axial length L1 of the end gap can be substantially equal to the maximum axial length L2 of the intermediate gap. The effect of easy expansion and deformation of the mesh opening on the radial side edge of the supporting cover 4 can be achieved as long as in the proximal cover 43, the first wave rod 4712 of the same hooking unit 47 close to the radial edge of the supporting cover 4 is configured to span over the top of the second wave rod 4722, and/or, in the distal cover 45, the second wave rod 4722 of the same hooking unit 47 close to the radial edge of the supporting cover 4 spans over the top of the first wave rod 4712.

Referring to FIG. 4, the side connecting members 46 are all connected to the hooking units 47. For example, the side connecting members 46 are respectively connected to two axially adjacent hooking units 47. Among the adjacent hooking units 47, the second wave rod 4722 in the second hook 472 of the hooking unit 47 closer to the proximal end of the concaved stent 100 continues to extend and bend to form a connecting hole 46a, and then is connected to the first wave rod 4712 of the first hook 471 of the hooking unit 47 closer to the distal end of the concaved stent 100. Both the first wave rod 4712 and the second wave rod 4722 are wave rods of the hooking unit 47 closer to the radial side edge of the supporting cover 4.

Referring to FIG. 4 and FIG. 8, in this embodiment, the supporting cover 4 further includes a side end connecting member 42a arranged at an axial end portion of the supporting cover 4. For example, both the distal end and the proximal end of the supporting cover 4 are provided with side end connecting members 42a which are configured to be connected to the edge of the concave 5. The side connecting member 46 is arranged between the side end connecting member 42a at the distal end of the supporting cover 4 and the side end connecting member 42a at the proximal end of the supporting cover 4, and the side end connecting member 42a is spaced apart from the side connecting members 46. By the arrangement of the side end connecting members 42a, the supporting cover 4 can better follow the bending deformation of the main stent 10, to better fit a blood vessel and support the concave 5. In addition, in this embodiment, each side end connecting member 42a includes an edge wave angle 421 that protrudes toward the axial end portion of the supporting cover 4, and is connected to the main stent 10 through the edge wave angle 421. For example, this connection may be a fixed connection by suturing, bonding, or the like. Since a vertex 4211 of the edge wave angle 421 itself does not form a closed connecting hole structure, this can reduce the radial contraction at an edge corner of the supporting cover 4, and can also form a larger polygonal mesh opening by the edge wave angle 421 and the hooking unit 47 adjacent to the edge wave angle 421. The polygonal mesh opening has a larger size, which is conducive to the entry of the guide wire and the external branch stent 300 from the mesh opening. In other embodiments, the side end connecting members 42a can be omitted.

When the maximum axial length of at least one end gap is greater than the maximum axial length of the middle interval, due to the connection between the side end connecting members 42a and the hooking units 47, the maximum distance between each side end connecting member 42a and the side connecting member 46 adjacent to it is also greater than the maximum distance between two adjacent side connecting members 46. This is conducive to increasing the size of the mesh opening on the side edge close to the axial end portion of the supporting cover 4 of the supporting cover 4, thus facilitating the entry of the guide wire and the external branch stent 300. In other embodiments, the maximum distance between each side end connecting member 42a and the side connecting member 46 adjacent to it may also be substantially equal to the maximum distance between two adjacent side connecting members 46.

In another embodiment, referring to FIG. 5, a plurality of side connecting members 46 include two first side connecting members 46a and a second side connecting member 46b located between the two first side connecting members 46a. The second side connecting member 46b is respectively connected to a first hook 471 and a second hook 472. The first side connecting members 46a are respectively connected to the side end connecting members 42a and the hooking unit 47 in the axial direction. For example, in the axial direction, the first side connecting member 46a close to the proximal end of the supporting cover 4 is respectively connected to the side end connecting member 42a located the proximal end of the supporting cover 4 and the first hook 471. For example, in the first hook 471 of the hooking unit 47 located at the proximal-most end, the first wave rod 4712 close to the radial edge of the supporting cover 4 continues to extend toward the proximal end of the supporting cover 4, then bends in reverse to form the first side connecting member 46a, and is connected to the side end connecting member 42a located at the proximal end of the supporting cover 4. In the axial direction, the first side connecting member 46a close to the distal end of the supporting cover 4 is respectively connected to the side end connecting member 42a located at the distal end of the supporting cover 4 and the second hook 472. For example, in the second hook 472 of the hooking unit 47 located at the distal-most end, the second wave rod 4722 (also refer to FIG. 4) close to the radial edge of the supporting cover 4 continues to extend toward the distal end of the supporting cover 4, then bends in reverse to form the first side connecting member 46a, and is connected to the side end connecting member 42a located at the distal end of the supporting cover 4. In this embodiment, each side end connecting member 42a includes an edge wave angle 421 that protrudes toward the axial end portion of the supporting cover 4. A polygonal mesh opening is formed by the edge wave angle 421 and the hooking unit 47 adjacent to the edge wave angle 421. The polygonal mesh opening has a larger size, which is conducive to the entry of the guide wire and the external branch stent 300 from the mesh opening. The side end connecting members 42a are connected to the first side connecting members 46a in this embodiment, and a distance exists between the first side connecting members 46a and the vertices 4211 of the edge wave angles 421 of the end connecting members. Therefore, this can increase the radial contraction size of the supporting cover 4 and can also ensure that larger mesh openings are formed between the side end connecting members 42a and the adjacent hooking unit 47, and prevent puncture and damage to a biological tissue because the edge wave angles 421 fixed by suturing move relative to the concave 5 when the supporting cover 4 is pressed or deforms.

In this embodiment, when the maximum axial length of at least one end gap is greater than the maximum axial length of the middle interval, the maximum distance between each first side connecting member 46a and the second side connecting member 46b adjacent to it is also greater than the maximum distance between two adjacent second side connecting members 46b. This is conducive to increasing the size of the mesh opening on the side edge close to the axial end portion of the supporting cover 4 of the supporting cover 4, thus facilitating the entry of the guide wire and the external branch stent 300. In other embodiments, the maximum distance between each side end connecting member 46a and the second side connecting member 46b adjacent to it may also be substantially equal to the maximum distance between two adjacent second side connecting members 46b.

Referring to FIG. 4, FIG. 8, and FIG. 12, the supporting cover 4 of this embodiment further includes a bending portion 422. The bending portion 422 includes a first rod 4222 and a second rod 4223 that are connected to each other. A bending angle 4221 is formed between the first rod 4222 and the second rod 4223. The first rod 4222 extends from the bending angle 4221 toward one axial end portion of the supporting cover 4, and the second rod 4223 extends from the bending angle 4221 toward another axial end portion of the supporting cover 4. Since the first rod 4222 and the second rod 4223 of the bending portion 422 respectively extend toward the opposite axial end portions of the supporting cover 4, with respect to a straight rod, when the supporting cover 4 is subjected to radial compression applied by a vascular wall and bends in accordance with the form of the blood vessel, the first rod 4222 and the second rod 4223 that form the bending angle 4221 can move relative to each other. Thus, the bending portion 422 can protrude away from the concave 5. The supporting cover 4 is more likely to form an arch in this region, to avoid compression of the space inside the concave 5 and facilitate the entry of the guide wire and the external branch stent 300 into the concave 5. Especially when the bending angle 4221 formed by the bending portion 422 is obtuse, the relative movement space between the first rod 4222 and the second rod 4223 is enlarged for more flexible deformation. Moreover, when the bending portion 422 protrudes away from the concave 5, formation of a sharp structure that may damage the vascular wall can be avoided.

In this embodiment, the bending angle 4221 of the bending portion 422 is located at the position close to the axial end portion of the supporting cover 4. For example, the bending angle 4221 is located between the axial end portion of the supporting cover 4 and the hooking unit 47 closest to the axial end portion. When an internal branch stent 6 is arranged near the axial end portion of the supporting cover 4, the bending portion 422 can form an arched structure that protrudes away from the concave 5 after the concaved stent 100 is implanted, to provide a larger space for the concave 5 at a branch opening of the internal branch stent 6 facing the concave 5. This facilitates the entry of the guide wire and the external branch stent 300 into the internal branch stent 6.

Further, the bending angle 4221 of the bending portion 422 bends toward the axial end portion of the supporting cover 4 close to the bending angle. This can make the mesh openings near the bending angle have more uniform sizes. In other embodiments, the bending angle 4221 of the bending portion 422 bends toward the axial end portion of the supporting cover 4 away from the bending angle.

The first rod 4222 of the bending portion 422 extends from the bending angle 4221 toward one axial end portion of the supporting cover 4, and the second rod 4223 extends from the bending angle 4221 toward another axial end portion of the supporting cover 4. Meanwhile, the first rod 4222 of the bending portion 422 extends toward one radial edge of the supporting cover 4, and the second rod 4223 extends toward another radial edge of the supporting cover 4. The advantage of this configuration is that both the first rod 4222 and the second rod 4223 extend obliquely in the axial and radial directions of the supporting cover 4, so that the bending portion 422 can better comply with the radial deformation of the supporting cover 4 and the axial bending deformation of the supporting cover 4.

One end away from the bending angle 4221 of the first rod 4222 of the bending portion 422 is connected to the edge of the concave 5. In this way, the edge of the concave 5 can provide a supporting force for the first rod 4222. When the concave 5 is subjected to a radial force, the end of the first rod 4222 away from the bending angle 4221 can better transmit the radial force, so that the first rod 4222 to more sensitively follow the deformation of the concave 5.

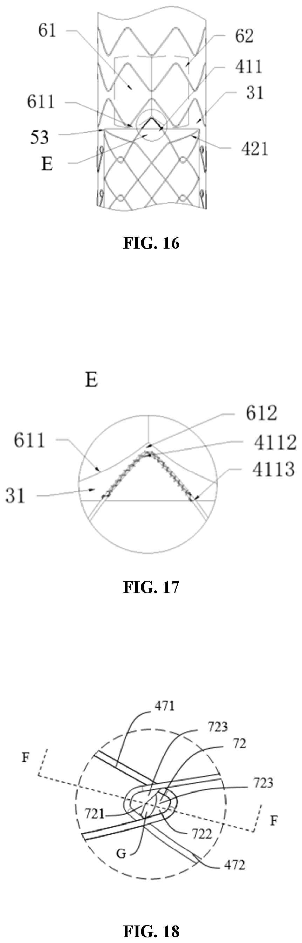

In this embodiment, referring to FIG. 4, FIG. 8, and FIG. 12, the edge wave angle 421 includes a vertex 4211 and two third wave rods 4212 connected to the vertex. The first rod 4222 serves as one of the third wave rods 4212 of the edge wave angle 421. Another third wave rod 4212 of the edge wave angle 421 extends from the vertex 4211 toward the axial end portion of the supporting cover 4 away from the vertex 4211 and is connected to the radial edge of the concave 5. Two third wave rods 4212 form an angle at the vertex 4211 of the edge wave angle 421. The angle formed at the vertex 4211 of the edge wave angle 421 should be appropriate. When the angle is too large, the sizes of the mesh openings on two sides of the first rod 4222 may be not uniform, and the first rod 4222 may substantially extend in the radial direction, making it difficult for sheath withdrawal for the concaved stent 100 to retract. When the angle is too small, the sizes of the mesh openings on two sides of the first rod 4222 may be not uniform, and the vertex 4211 of the edge wave angle 421 may easily puncture and injure a blood vessel. Therefore, the angle may be acute. For example, the angle may range from 30° to 70°. Within this range, the sizes of the mesh openings on the two sides of the first rod 4222 may be uniform, and the vertex can also facilitate the sheath withdrawal for the concaved stent 100. It has good safety. The edge wave angle 421 is connected to an edge corner 53 of the concave 5 (refer to FIG. 16). For example, the vertex 4211 of the edge wave angle 421 is connected to the edge corner 53 of the concave 5. Therefore, an effect of supporting the edge corner 53 of the concave 5 is achieved to an extent, so that the edge corner 53 of the concave 5 is fully unfolded, and the form of the opening of the concave 5 can be better maintained.

The second rod 4223 of the bending portion 422 passes through the second mesh region 42 and is connected to the vertex of the first hook 471 or the second hook 472 on the opposite side. For example, a portion of the second rod 4223 serves as a supporting wire of the second mesh region 42, and a portion serves as a wave rod of the hooking unit. In this way, the vertex of the first hook 471 or the second hook 472 provides a supporting force to the second rod 4223. When the concave 5 and the supporting cover 4 are subjected to a radial force, the end of the second rod 4223 away from the bending angle 4221 can better transmit the radial force, so that the second rod 4223 can more flexibly follow the deformation of the supporting cover 4. In addition, the second rod 4223 reaches the opposite side through the second mesh region 42, so the first rod 4222 and the second rod 4223 can respectively transmit the compressive force on the two radial sides of the supporting cover 4, thereby adaptively undergoing deformation and effectively maintaining the space inside the concave 5.

In this embodiment, the supporting cover 4 includes four side end connecting members 42a and four bending portions 422. Two side end connecting members 42a and two bending portions 422 are located at the proximal end of the supporting cover 4. The two side end connecting members 42a are respectively connected to two edge corners 53 of the proximal end of the concave 5, and the two bending portions 422 are respectively connected to the two side end connecting members 42a. Two side end connecting members 42a and two bending portions 422 are located at the distal end of the supporting cover 4. The two side end connecting members 42a are respectively connected to two edge corners 53 of the distal end of the concave 5, and the two bending portions 422 are respectively connected to the two side end connecting members 42a. In other embodiments, the number of side end connecting members 42a and the number of bending portions can be selected according to an actual application scenario.

Referring to FIG. 5, in another embodiment, the supporting cover 4 further includes a side end connecting member 42a and an intermediate end connecting member 41a that are connected to the main stent 10. The side end connecting member 42a is closer to the radial edge of the supporting cover 4 than the intermediate end connecting member 41a. Compared with the solution in which the intermediate end connecting member 41a is not connected to the main stent, this solution indicates that the intermediate end connecting member 41a is connected to the main stent 10. Therefore, when the main stent 10 bends, it can more effectively drive the supporting cover 4 to undergo bending deformation in accordance with a form of a blood vessel. Furthermore, the intermediate end connecting member 41a connected to the main stent 10 can also provide a better supporting force for an axial end portion of the concave 5, to avoid the formation of a gap between the main stent 10 and the intermediate end connecting member 41a in the axial direction when the concaved stent 100 bends, thereby avoiding situations where an inner wall of a blood vessel with a narrow true lumen compresses the gap and enters an internal space of the concave 5 from the gap, which could obstruct the guide wire and the external branch stent 300 from entering the internal branch stent 6.

Further, the intermediate end connecting member 41a located at the proximal end of the supporting cover 4 is closer to the proximal end of the concaved stent 100 than the side end connecting member 42a located at the proximal end of the supporting cover 4, and/or the intermediate end connecting member 41a located at the distal end of the supporting cover 4 is closer to the distal end of the concaved stent 100 than the side end connecting member 42a located at the distal end of the supporting cover 4. Compared with the solution in which an axial end portion of the side end connecting member 42a is flush with an axial end portion of the intermediate end connecting member 41a, the solution of this embodiment allows the supporting cover 4 at the position of the intermediate end connecting member 41a to have a larger axial size, so that the supporting cover 4 can form a better arched structure after bending along with the main stent 10. This can effectively prevent the axial end portions of the supporting cover 4 from forming an approximate plane when the main stent 10 bends, better maintain the internal space of the concave 5, and facilitate the entry of the guide wire and the external branch stent 300 into the concave. In addition, after the external branch stent 300 is implanted, excessive compression on the external branch stent 300 is avoided.

For example, referring to FIG. 5 and FIG. 16, the intermediate end connecting member 41a includes an intermediate wave angle 411. The side end connecting member 42a includes an edge wave angle 421. A vertex 4112 of an intermediate wave angle 411 located at the proximal end of the supporting cover 4 is closer to the proximal end of the concaved stent 100 than the vertex 4211 of the edge wave angle 421, and a vertex 4112 of an intermediate wave angle 411 located at the distal end of the supporting cover 4 is closer to the distal end of the concaved stent 100 than the vertex 4211 of the edge wave angle 421. Compared with the solution in which the vertex 4112 of the intermediate wave angle 411 is flush with the vertex 4211 of the edge wave angle 421 (refer to FIG. 4), after the supporting cover 4 bends, due to a greater protrusion length of the intermediate wave angle 411, the stretching degree of the edge wave angles 421 on the two sides in the axial direction is smaller, which can better maintain the shapes and sizes of the mesh openings in regions in which the edge wave angles 421 are located and facilitates the guide wire and the external branch stent 300 to pass through these mesh openings. Further, the intermediate wave angle 411 in the middle provides a sufficient stretching length to ensure that the proximal end and distal end of the supporting cover 4 do not stretch and deform too much toward the inner cavity of the concave 5. This can effectively prevent the axial end portions of the supporting cover 4 from forming an approximate plane when the main stent 10 bends, but instead forming a good arched structure, thereby effectively maintaining the internal space formed by the proximal end and distal end of the supporting cover 4 and the concave 5.

In other embodiments, the vertex 4112 of the intermediate wave angle 411 shown in FIG. 4 is similarly flush with the vertex 4211 of the edge wave angle 421. Or, the vertex 4211 of the edge wave angle 421 is closer to the axial end portion of the corresponding concaved stent 100 than the vertex 4112 of the intermediate wave angle 411.

In one embodiment, as shown in FIG. 5, FIG. 16, and FIG. 17, the intermediate wave angle 411 includes two fourth wave rods 4113 connected to the vertex 4112 of the intermediate wave angle 411. The vertex 4112 of the intermediate wave angle 411 is connected to one axial end portion of the concave 5, and the two fourth wave rods 4113 extend from the vertex 4112 of the intermediate wave angle 411 toward another axial end portion of the concave 5. Further, the vertex 4112 of the intermediate wave angle 411 is axially opposite to the second mesh region 42, and two fourth wave rods 4113 extend from the vertex 4112 of the intermediate wave angle 411 away from each other, so as to be connected to the first mesh regions 41 on the two radial sides of the second mesh region 42; for example, to the hooking units 47 on the two radial sides of the supporting cover 4. When the main stent 10 protrudes and bends toward the opening of the concave 5, the intermediate wave angle 411 can drive the first mesh regions 41 connected to the intermediate wave angle to bend, and at the same time, drive the second mesh region 42 connected to the first mesh regions 41 to bend. Further, since the first mesh regions 41 include a plurality of hooking units 47, when the intermediate wave angle 411 is connected to the hooking units 47, the mesh openings of the first mesh regions 41 can be driven to fully expand during bending, making it more convenient to thread the guide wire and the external branch stent 300. Meanwhile, the hooking units 47 can also limit stretching lengths of the first mesh regions 41 to an extent, thereby limiting the stretching length of the second mesh region 42 connected to the first mesh regions to avoid the problems such as insufficient compression of the internal space of the compression concave 5, insufficient shrinkage of the mesh openings of the second mesh region 42, and the like due to excessive stretching of the second mesh region 42. Therefore, it can better maintain the internal space of the concave 5 and the forms of the mesh openings of the second mesh region 42, thus further facilitating the guide wire and the external branch stent 300 to be threaded.

Referring to FIG. 5, FIG. 16, and FIG. 17, the two fourth wave rods 4113 of the intermediate wave angle 411 located at the proximal end of the supporting cover 4 extend from the vertex 4112 of the intermediate wave angle 411 away from each other to form the first wave rods 4712 of the two hooking units 47 on the two radial sides of the supporting cover 4. The two fourth wave rods 4113 of the intermediate wave angle 411 located at the distal end of the supporting cover 4 extend from the vertex 4112 of the intermediate wave angle 411 away from each other to form the second wave rods 4722 of the two hooking units 47 on the two radial sides of the supporting cover 4. The two fourth wave rods 4113 of the intermediate wave angle 411 can be substantially parallel to the first direction supporting wire 481 and the second direction supporting wire 482 of the second mesh region 42, respectively. In other embodiments, the two fourth wave rods 4113 of the intermediate wave angle 411 may not be parallel to the first direction supporting wire and the second direction supporting wire of the second mesh region 42. An angle of the intermediate wave angle 411 (i.e. the angle formed by the two fourth wave rods 4113 at the vertex 4112 of the intermediate wave angle 411) should be appropriate. When the intermediate wave angle 411 is too large and is connected to the main covering film 31 by suturing, the intermediate wave angle 411 may easily slide relative to the main covering film 31 in the longitudinal directions of the fourth wave rods 4113. When the angle of the intermediate wave angle 411 is too small, the intermediate wave angle 411 easily punctures the main covering film 311 and damages a vascular wall. Thus, the angle of the intermediate wave angle 411 may range from 20° to 80°. On the one hand, the intermediate wave angle 411 can be stably fixed, and on the other hand, the intermediate wave angle 411 can be prevented from puncturing the main covering film 31 and damaging the vascular wall.

Further, referring to FIG. 5 and FIG. 17, the two fourth wave rods 4113 of the intermediate wave angle 411 respectively span over the tops of the bending portions 422. In this embodiment, the two fourth wave rods 4113 of the intermediate wave angle 411 respectively span over the tops of the second rods 4223 of two bending portions 422. In other embodiments, the two fourth wave rods 4113 of the intermediate wave angle may also span over the tops of the first rods 4222 of two bending portions 422, as long as the two fourth wave rods 4113 of the intermediate wave angle 411 respectively span over the tops of the bending portions 422. The advantage of this configuration is that when the supporting cover 4 is subjected to radial compression or bending, the bending portions 422 can protrude away from the concave 5, thereby jacking the fourth wave rods 4113 spanning over the tops of the bending portions. This prevents formation of a flat structure because of the excessive stretching of the intermediate wave angle 411 after the concaved stent 100 bends. Therefore, the axial end portions of the supporting cover 4 can better maintain the internal space of the concave 5, to avoid occupation of a space near the opening of the internal branch stent 6.

When the concaved stent 100 is in an unfolded state, the fourth wave rods 4113 of the intermediate wave angle 411 can be in contact with the bending portions 422. Or, gaps exist between the fourth wave rods 4113 of the intermediate wave angle 411 and the bending portions 422 in the radial direction of the concaved stent 100. In spite of mutual contact or the formation of the gaps, the intermediate wave angle 411 can move relative to the bending portions 422, thereby maintaining better deformation capability and compliance whether the supporting cover 4 bends or is in a straight state. This can better maintain the internal space formed between the supporting cover 4 and the concave 5. When the gaps exist between the fourth wave rods 4113 of the intermediate wave angle 411 and the bending portions 422 in the radial direction of the concaved stent 100, the formed gaps can enable the bending portions 422 to have a larger movement space. This facilitates better formation of the arched structure when the supporting cover 4 bends away from the concave 5, and is more conducive for the guide wire and the external branch stent 300 to enter the internal branch stent 6.

In one embodiment, the fourth wave rods 4113 of the intermediate wave angle 411 may be arc-shaped rods, and the arc-shaped rods are of upwards convex arched bending structures. Specifically, the upward convexity here refers to a protrusion away from a central axis of the concaved stent 100 in the radial direction of the concaved stent 100. This convex arched bending structure can be more conductive to forming the gaps between the intermediate wave angle 411 and the bending portions 422. Further, due to the arc-shaped rods with the arched bending structures, after the supporting cover 4 axially bends along with the bending of the main stent 10, the axial end portions of the supporting cover 4 can form the arched structure along the arc-shaped rods with the arched bending structures, thereby providing higher supporting performance. This can better maintain the internal space of the concave 5 at the axial end portion and avoid excessive compression on the internal space.

Referring to FIG. 16 and FIG. 17, the internal branch stent 6 of the main stent 10 located at the proximal section 2 is a dual-branch-opening stent 61. The dual-branch-opening stent 61 is composed of two single-branch-opening stents 62 connected side by side to an inner wall of the proximal section 2 of the concaved stent 100 via suturing or bonding. The two single-branch-opening stents 62 are typically configured as substantially circular branch openings, and a gap position 612 can be formed between the two branch openings. When the dual-branch-opening stent 61 is sutured at a proximal edge of the concave 5 to a proximal main covering film of the concaved stent 100, a blank section of the proximal main covering film can be formed at the gap position 612. When the supporting cover 4 is mounted in the concave 5, the intermediate end connecting member 41a of the supporting cover 4 is connected to the proximal main covering film at the gap position 612. For example, the vertex 4112 of the intermediate wave angle 411 extends to the gap position 612, so that the vertex 4112 of the intermediate wave angle 411 is closer to the proximal end of the concaved stent 100 than the proximal edge of the concave 5, and the intermediate wave angle 411 is connected to the proximal main covering film at the gap position 612. The advantage of this configuration is that branch openings 611 of the dual-branch-opening stent 61 can play a role of supporting the intermediate wave angle 411 and prevent the intermediate wave angle 411 from puncturing the main covering film 31.

It can be understood that the above intermediate end connecting member 41a can be connected to an inner wall or an outer wall of the main covering film 31 (refer to FIG. 2). For example, the vertex 4112 of the intermediate wave angle 411 and part of each fourth wave rod 4113 extend to the inner wall of the main covering film 31 at the gap position 612, namely, the main covering film 31 at the gap position 612 covers the vertex 4112 of the intermediate wave angle 411 and an outer side of part of each fourth wave rod 4113, and the vertex 4112 of the intermediate wave angle 411 and part of each fourth wave rod 4113 are connected to the inner wall of the main covering film 31 via suturing. Alternatively, the main covering film 31 at the gap position 612 covers the vertex 4112 of the intermediate wave angle 411 and an inner side of part of each fourth wave rod 4113, and the vertex 4112 of the intermediate wave angle 411 and part of each fourth wave rod 4113 are connected to the outer wall of the main covering film 31 via suturing. When the intermediate end connecting member 41a is connected to an inner wall of the main covering film 31, the intermediate end connecting member 41a can be prevented from upwarping outwards and damaging an inner wall of a blood vessel when the concaved stent 100 deforms, thereby improving the safety performance.

In other embodiments, the internal branch stent 6 of the main stent 10 located at the proximal section 2 may not be a dual-branch-opening stent 61, while the internal branch stent 6 at the distal section 2 is a dual-branch-opening stent 61. In this way, the intermediate wave angle 411 located at the distal end of the supporting cover 4 can be connected to the main stent 10 based on the above connecting manner. In other embodiments, the connecting position and connection for the intermediate wave angle 411 and the main support 10 are not limited to the above, and appropriate connecting positions and connections can be selected based on actual needs.

Further, a supporting member is further arranged at an edge of each branch opening 611 of the dual-branch-opening stent 61, to better maintain the form of the branch opening 611. At least a portion of the supporting member is connected to the main covering film 31 and is located on two sides of the intermediate end connecting member 41a to provide a better support for the intermediate wave angle 411.

### Second embodiment

The concaved stent 100 of this embodiment is substantially the same as the first embodiment, and identical parts will not be elaborated. A difference lies in the structure of the protective unit 72 of the concaved stent 100 of this embodiment.

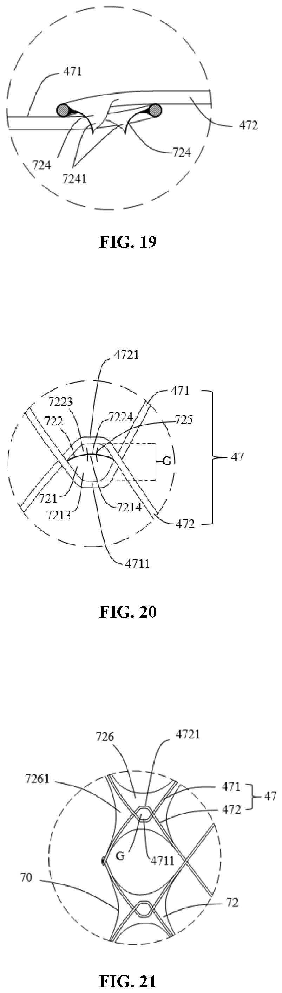

Referring to FIG. 18 and FIG. 19, at least one of the first protective unit 721 and the second protective unit 722 of this embodiment includes a main sheet 723 and an extension sheet 724. Both the main sheet 723 and the extension sheet 724 are sheet-like structures. One end of the main sheet 723 is connected to the hooking unit 47, and another end extends into the hooking gap G and is connected to the extension sheet 724. The extension sheet 724 has a free end 7241 and extends toward the concave bottom 51. The free end 7241 of the extension sheet 724 is located between the supporting cover 4 and the concave bottom 51. The arrangement of the extension sheet 724 is beneficial for enlarging the contact area between the protective unit 72 and the external branch stent 300 inserted thereto, thereby better protecting the external branch stent 300. In addition, the extension sheet 724 extends into the radial gap between the concave bottom 51 and the supporting cover 4. This will alter the hemodynamics in the concave 5, thereby accelerating thrombogenesis within a short period of time and making a thrombus quickly fill the concave 5. Thus, a sealing effect is achieved on the concave 5, which is conductive to supporting the implanted external branch stent 300 and preventing distortion and displacement of the external branch stent 300. This is also conducive to rapid endothelialization of the concave 5 and the supporting cover 4, thus further lowering the risk of damage to the external branch stent 300 caused by the frictional force and the cutting force.

In this embodiment, the main sheet 723 and the extension sheet 724 may be made of one or more of the following polymer materials: PTFE, PET, ePTFE, FEP, silica gel, L-polylactic acid, racemic polylactic acid, polyglycolic acid, a polylactic acid-hydroxyacetic acid copolymer, polyhydroxyalkanoate, polydioxanone, polycaprolactone, polyglucose acid, polyhydroxybutyrate, polyanhydride, polyphosphate ester, polyglycolic acid, and polydioxanone. The main sheet 723 and the extension sheet 724 can be made of the same material or different materials.

Further, the extension sheet 724 may have a guide function, which can bend toward the internal branch stent 6 to guide the guide wire to better enter the internal branch stent 6. This is conducive to improving the efficiency and success rate of surgery. For example, the extension sheet 724 may be made of a silica gel material, which has shape stability and can effectively guide the guide wire to move toward the internal branch stent 6. In another embodiment, each of the first protective unit 721 and the second protective unit 722 includes an extension sheet 724, and the extension sheets 724 of the two protective units are at least partially connected to each other to form a guide channel that bends and extends toward the internal branch stent 6, thereby more effectively guiding and promoting endothelialization.

### Third embodiment

The concaved stent 100 of this embodiment is substantially the same as the first embodiment, and identical parts will not be elaborated. A difference lies in the structure of the protective unit 72 of the concaved stent 100 of this embodiment.

Referring to FIG. 20, at least one of the first protective unit 721 and the second protective unit 722 includes a sheet-like structure. The sheet-like structure has a movable end. At least a portion of the movable end can move relative to the hooking unit 47. The movable end includes a thin-walled region. The thickness of the thin-walled region is less than the thickness of another region of the protective unit 72. When the external branch stent 300 is inserted into the hooking gap G from the outside toward the concave 5, at least a portion of the thin-walled region deforms relative to another region and extends toward the concave bottom 51, thus forming a structure similar to the extension sheet 724 in the second embodiment. This is beneficial for enlarging the contact area between the protective unit 72 and the external branch stent 300 inserted thereto, thereby better protecting the external branch stent 300. In addition, the portion of the thin-walled region extending between the concave bottom 51 and the supporting cover 4 alters the hemodynamics in the concave 5, thereby accelerating thrombogenesis within a short period of time and making a thrombus quickly fill the concave 5. Thus, a sealing effect is achieved on the concave 5, which is conducive to supporting the implanted external branch stent 300 and preventing distortion and displacement of the external branch stent 300. This is also conducive to rapid endothelialization of the concave 5 and the supporting cover 4, thus further lowering the risk of damage to the external branch stent 300 caused by the frictional force and the cutting force.

In this embodiment, referring to FIG. 21, the first protective unit 721 includes a third sheet-like structure 7213, and the second protective unit 722 includes a fourth sheet-like structure 7223. The third sheet-like structure 7213 has a first movable end 7214, and the first movable end 7214 includes a thin-walled region. An edge of the first movable end 7214 is an edge of one end of the third sheet-like structure 7213 farther away from the valley 4711. The edge of the first movable end 7214 is substantially in an arc shape that bends toward the peak 4721. The fourth sheet-like structure 7223 has a second movable end 7224, and the second movable end 7224 includes a thin-walled region. An edge of the second movable end 7224 is an edge of one end of the fourth sheet-like structure 7223 farther away from the peak 4721. The edge of the second movable end 7224 is substantially in an arc shape that bends toward the peak 4721. In addition, the shape of the edge of the second movable end 7224 matches the shape of the edge of the first movable end 7214, to jointly completely shield the hooking gap G. Through this configuration, when the external branch stent 300 is inserted between the third sheet-like structure 7213 and the fourth sheet-like structure 7223, the thin-walled regions of the first movable end 7214 and the second movable end 7224 are more likely to deform relative to other regions of the sheet-like structures and extend toward the concave bottom 51. It can be understood that in other embodiments, the edge of the first movable end 7214 and the edge of the second movable end 7224 can be in any other suitable shapes, as long as their shapes match each other and jointly completely shield the hooking gap G. For example, the two edges are in a straight-line shape, an S shape, a zigzag shape, or the like. In other embodiments, the thin-walled region of the first movable end 7214 and the thin-walled region of the second movable end 7224 may overlap in the radial direction of the concave 5 to jointly completely shield the hooking gap G. In this case, the edge of the first movable end 7214 and the edge of the second movable end 7224 may be in any suitable shape and may not necessarily match each other.

Further, in other embodiments, the thin-walled regions can be made closer to the concave bottom 51 relative to the hooking unit 47, which can increase the probability of deformation of the thin-walled regions relative to other regions of the sheet-like structures when the external branch stent 300 is inserted into the hooking gap G. In addition, other regions of the sheet-like structures can play a role in guiding.

Further, referring to FIG. 20, each thin-walled region may include at least one crack-prone strip 725. The crack-prone strip 725 may be in any suitable shape such as a straight-line shape, an S shape, or a zigzag shape. In this embodiment, the crack-prone strip 725 may be a region that is thinner than another region of the thin-walled region, for example, an indentation obtained by hot pressing on the thin-walled region. A region in which the indentation is located is thinner than another region of the thin-walled region. In other embodiments, a plurality of small holes may be formed at intervals in a length direction of the crack-prone strip 725, which can also achieve an easy cracking effect.

In this embodiment, the crack-prone strip 725 extends substantially in the axial direction of the concaved stent 100, with one end extending to an edge of the movable end and another end extending away from the movable end. In other embodiments, the crack-prone strip 725 may be inclined at an angle to the axial direction of the concaved stent 100, and the angle may be greater than 0° and less than 90°. In other embodiments, the crack-prone strip 725 may not extend to the edge of the movable end and may form 90° to the axial direction of the concaved stent 100. When the crack-prone strip 725 forms a crack, the sheet-like structure is divided into at least two pieces in the axial direction. One piece closer to the edge of the movable end is more easily driven by the inserted external branch stent 300 to deform toward the concave bottom 51.

The crack-prone strip 725 of this embodiment cracks to form a crack when the external branch stent 300 is inserted into the hooking gap G from the outside toward the concave bottom 51; and at least a portion adjacent to the crack of the thin-walled region deforms relative to another region of the sheet-like structure and extends toward the concave bottom 51. This configuration is conducive to further increasing the probability of deformation of the thin-walled region relative to another region of the sheet-like structure when the external branch stent 300 is inserted into the hooking gap G, and the crack-prone strip can more effectively guide the thin-walled region to form a desired shape after the external branch stent 300 is inserted.

The protective unit 72 may be made of one or more of the following polymer materials: PTFE, PET, ePTFE, FEP, silica gel, L-polylactic acid, racemic polylactic acid, polyglycolic acid, a polylactic acid-hydroxyacetic acid copolymer, polyhydroxyalkanoate, polydioxanone, polycaprolactone, polyglucose acid, polyhydroxybutyrate, polyanhydride, polyphosphate ester, polyglycolic acid, and polydioxanone.

### Fourth embodiment

The concaved stent 100 of this embodiment is substantially the same as the first embodiment, and identical parts will not be elaborated. A difference lies in the structure of the protective unit 72 of the concaved stent 100 of this embodiment.

Referring to FIG. 21, the protective structure 70 of this embodiment includes a third protective unit 726. The third protective unit 726 includes a polymer covering film 7261 covering the hooking gap G, and the valley 4711 of the first hooking unit 471 and/or the peak 4721 of the second hooking unit 472 can move relative to each other in the axial direction. An insertion hole is formed in at least a portion of the supporting cover 4. For example, an insertion hole is formed between two adjacent hooking units 47 for inserting the guide wire and the external branch stent 300. This configuration is conducive to reducing the probability that the external branch stent 300 is guided by the guide wire to enter the hooking gap G, thereby reducing the probability of frictional damage to the external branch stent 300.

The third protective unit 726 includes a double-layer polymer covering film, namely a first covering film and a second covering film. The first covering film shields the hooking gap G on the outer side (i.e. a side farther away from the concave bottom 51) of the hooking unit 47, and the second covering film shields the hooking gap G on the inner side (i.e. a side closer to the concave bottom 51) of the hooking unit 47. The first covering film and the second covering film overlap to jointly sandwich the hooking unit 47 between them. An accommodating gap is further formed between the first covering film and the second covering film, and the valley 4711 of the first hooking unit 471 and/or the peak 4721 of the second hooking unit 472 are located within the accommodating gap, so that the valley 4711 of the first hooking unit 471 and/or the peak 4721 of the second hooking unit 472 can move relative to each other in the axial direction, and the supporting cover 4 still has good bending performance, which can effectively deform by conforming to the form of a vascular wall.

In other embodiments, the valley 4711 of the first hooking unit 471 and/or the peak 4721 of the second hooking unit 472 may be located on one side of the double-layer polymer covering film. For example, the first covering film is provided with a first via and a second via. The valley 4711 of the first hooking unit 471 and part of a wave rod section connected to the valley 4711 are threaded out from the first via and are located on an outer side of the first covering film. The peak 4721 of the second hooking unit 472 and part of a wave rod section connected to the peak 4721 protrude from the second via and are also located on the outer side of the first covering film. In other embodiments, the second covering film is provided with a first via and a second via. The valley 4711 of the first hooking unit 471 and part of a wave rod section connected to the valley 4711 are threaded out from the first via and are located on an inner side of the second covering film. The peak 4721 of the second hooking unit 472 and part of a wave rod section connected to the peak 4721 protrude from the second via and are also located on the inner side of the second covering film. In other embodiments, the valley 4711 of the first hooking unit 471 and part of the wave rod section connected to the valley 4711 may be located on the outer side of the first covering film, while the peak 4721 of the second hooking unit 472 and part of the wave rod section connected to the peak 4721 may be located on the inner side of the second covering film. Alternatively, the valley 4711 of the first hooking unit 471 and part of the wave rod section connected to the valley 4711 are located on the inner side of the second covering film, while the peak 4721 of the second hooking unit 472 and part of the wave rod section connected to the peak 4721 are located on the outer side of the first covering film, as long as the polymer covering film 7261 can completely cover the hooking gap G, and the valley 4711 of the first hooking unit 471 and/or the peak 4721 of the second hooking unit 472 can move relative to each other in the axial direction.

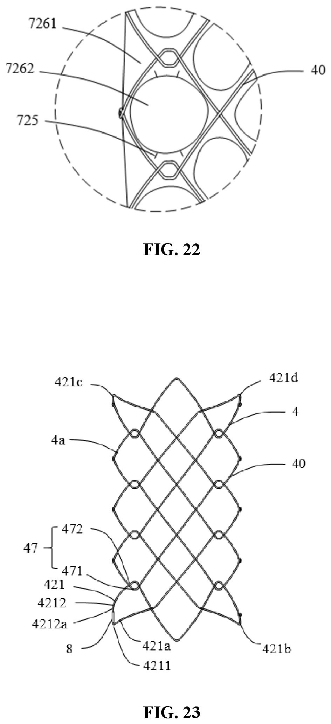

Referring to FIG. 22, the polymer covering film 7261 can further cover other braiding wires 40 on the supporting cover 4 to further provide better protection for the supporting cover 4 and the external branch stent 300. However, an insertion hole 7262 needs to be reserved in at least a portion of the supporting cover 4 to allow the guide wire and the external branch stent 300 to be inserted into the radial gap between the supporting cover 4 and the concave bottom 51 through the insertion hole 7262. The shape of the insertion hole 7262 is not limited. For example, it can be a circle, an ellipse, a triangle, a quadrangle, or another polygon, or any other suitable shape.

Further, one or more crack-prone strips 725 can be arranged at an edge of the insertion hole 7262, and the shape and position of each crack-prone strip 725 can be found in the description of the third embodiment, and will not be elaborated here. Under an external force, each crack-prone strip 725 easily cracks to increase a perimeter of the insertion hole 7262. This can adapt to the insertion of external branch stents 300 of various sizes. After an external branch stent 300 is inserted, a region of the polymer covering film 7261 adjacent to the crack formed by the crack-prone strip 725 deforms relative to another region of the polymer covering film 7261 and extends toward the concave bottom 51. This is beneficial for enlarging the contact area between the protective unit 72 and the external branch stent 300 inserted thereto, thereby better protecting the external branch stent 300. In addition, the region of the polymer covering film 7261 extending between the concave bottom 51 and the supporting cover 4 alters the hemodynamics in the concave 5, thereby accelerating thrombogenesis within a short period of time and making a thrombus quickly fill the concave 5. Thus, a sealing effect is achieved on the concave 5, which is conducive to supporting the implanted external branch stent 300 and preventing distortion and displacement of the external branch stent 300. This is also conducive to rapid endothelialization of the concave 5 and the supporting cover 4, thus further lowering the risk of damage to the external branch stent 300 caused by the frictional force and the cutting force.

The protective unit 72 may be made of one or more of the following polymer materials: PTFE, PET, ePTFE, FEP, silica gel, L-polylactic acid, racemic polylactic acid, polyglycolic acid, a polylactic acid-hydroxyacetic acid copolymer, polyhydroxyalkanoate, polydioxanone, polycaprolactone, polyglucose acid, polyhydroxybutyrate, polyanhydride, polyphosphate ester, polyglycolic acid, and polydioxanone.

### Fifth embodiment

This embodiment provides a concaved stent 100. The concaved stent 100 includes a main stent 10 and a supporting cover 4. The main stent 10 is tubular and has an inner cavity. A side surface of the main stent 10 is recessed toward the inner cavity to form a concave 5. The concave 5 includes a concave bottom 51. The supporting cover 4 is connected to the main stent 10, and at least a portion of the supporting cover 4 and the concave bottom 51 form a radial gap in the radial direction of the concaved stent 100. The supporting cover 4 includes a mesh structure 4a braided from a braiding wire 40. The mesh structure 4a includes a plurality of mesh openings. The concave 5 communicates with the outside via the mesh openings. The concaved stent 100 of this embodiment can adopt any of the stent structures described in the first embodiment to the fourth embodiment. The specific stent structure can be found in the content described in the first embodiment to the fourth embodiment, which will not be elaborated in this embodiment. It can be understood that this embodiment can also adopt any other suitable stent structures. For example, a braided structure of the supporting cover 4 of the concaved stent 100 may be different from those in the previous embodiments, and the supporting cover 4 may not be provided with a protective structure 70. In the foregoing embodiments, the supporting cover 4 of the concaved stent 100 includes the mesh structure 4a braided from the braiding wire 40. The mesh structure 4a includes a braiding starting end B0 and a braiding tail end E0. The braiding starting end B0 and the braiding tail end E0 may be connected to each other via welding, bonding, winding, or the like, or may be fixedly or movably connected.

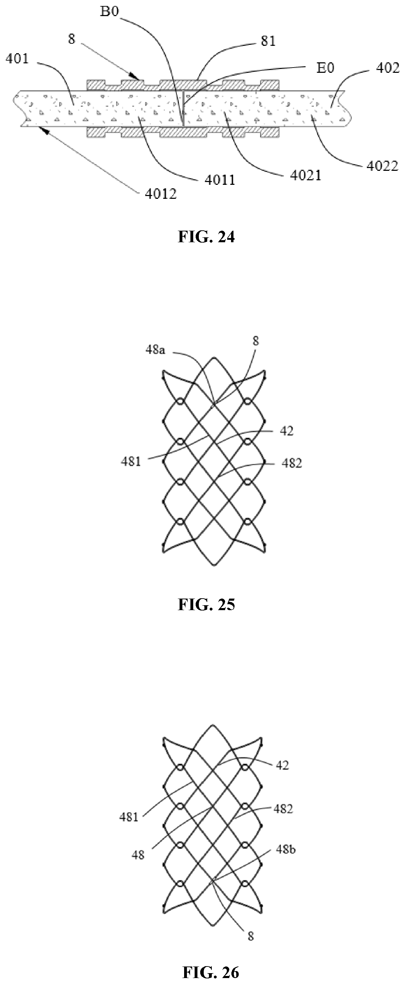

Referring to FIG. 23 and FIG. 24, the braiding starting end B0 and the braiding tail end E0 are fixed to each other through a fixing member 8. The fixing member 8 not only stably connects the braiding starting end B0 to the braiding tail end E0, but also avoids the risk of mechanical property damage to regions near the braiding starting end B0 and the braiding tail end E0 caused by welding.

For example, the fixing member 8 includes a fixing sleeve 81. The fixing sleeve 81 includes a sleeve inner cavity, and the braiding starting end B0 and the braiding tail end E0 are fixed in the sleeve inner cavity. The braiding starting end B0 and the braiding tail end E0 can be fixed to the fixing sleeve 81 by applying a pressure to the exterior of the fixing sleeve 81. The braiding starting end B0 and the braiding tail end E0 are opposite to each other in a longitudinal direction of the braiding wire 40. For example, the braiding starting end B0 and the braiding tail end E0 are abutted with each other shown in the figure. In other embodiments, the braiding starting end B0 and the braiding tail end E0 may be spaced apart from each other. The advantage of making the braiding starting end B0 and the braiding tail end E0 opposite to each other in the longitudinal direction in the fixing sleeve 81 is that the width of the connection position between the braiding starting end B0 and the braiding tail end E0 can be reduced, so that a fixing sleeve 81 with a smaller width can be used. This can lower the risk that the insertion of the guide wire or the external branch stent 300 is obstructed because the mesh openings adjacent to the fixing member 8 cannot be fully unfolded since the fixing sleeve 81 hooks the braiding wire 40 in other regions. In addition, the risk of damage to the braiding wire 40 caused by the fixing member 8 rubbing and hooking the braiding wire 40 in other regions can also be lowered. It can be understood that in other embodiments, the braiding starting end B0 and the braiding tail end E0 can also be arranged side by side in a width direction in the fixing sleeve 81. It can be understood that the structure of the fixing member 8 in this embodiment is not unique. In addition to the fixing sleeve 81 illustrated in this embodiment, it can also be a fixing wire or a fixing ring. For example, the braiding starting end B0 and braiding tail end E0 can be fixed through a fixing wire via winding or suturing, or the braiding starting end B0 and the braiding tail end E0 can be fixed to a fixing ring via winding.

The fixing member 8 can be made of a material with an imaging function. The fixing member 8 can assist in positioning under imaging equipment such as digital subtraction angiography (DSA). For example, the fixing member 8 can be made of a metal material such as stainless steel, nickel titanium, or platinum. Or, the fixing member 8 can also be made of a polymer material doped with a developing material such as iohexol, or may be made of any other suitable materials. When the fixing member 8 includes the fixing sleeve 81, since the fixing sleeve 81 is surrounds the braiding starting end B0 and braiding tail end E0, the width of the fixing sleeve is greater than the wire diameter of each braiding wire 40. Therefore, the position of the fixing member 8 can be effectively distinguished in an image, which is conducive to enhancing an auxiliary positioning effect.

In this embodiment, a plurality of fixing members 8 may be provided. The axial distance between at least one fixing member 8 and the proximal end of the concave opening 52 is less than the axial distance between the fixing member 8 and the distal end of the concave opening 52. Alternatively, the axial distance between at least one fixing member 8 and the proximal end of the concave opening 52 is greater than the axial distance between the fixing member 8 and the distal end of the concave opening 52. The purpose of this configuration is to instruct a surgical operator to align a branch blood vessel 200, so that a subsequent insertion position of the external branch stent 300 is more accurate. In addition, through this configuration, after the concaved stent 100 is implanted, the risk of damage to the fixing member 8 and the braiding wire 40 adjacent to the fixing member 8 caused by protruding bending and lateral bending of the concaved stent 100 toward the supporting cover 4 because of compliance with a blood vessel is lowered.

For example, when the axial distance between the fixing member 8 and the proximal end of the concave opening 52 is less than the axial distance between the fixing member 8 and the distal end of the concave opening 52, the ratio of the axial distance between the fixing member 8 and the proximal end of the concave opening 52 to the axial length of the concave opening 52 ranges from 5% to 15%. When the axial distance between the fixing member 8 and the proximal end of the concave opening 52 is greater than the axial distance between the fixing member 8 and the distal end of the concave opening 52, the ratio of the axial distance between the fixing member 8 and the distal end of the concave opening 52 to the axial length of the concave opening 52 ranges from 5% to 15%. Due to the continuous pulsation of a blood vessel, the implanted concaved stent 100 may have slight displacement. This may cause a difference between the position indicated by the fixing member 8 and the expected position. By limiting the axial distance between the fixing member 8 and the axial end portion of the concave opening 52 to be greater than or equal to 5% of the axial length of the concave opening 52, a positional error can be reserved for the displacement of the concaved stent 100 caused by the pulsation of the blood vessel. In addition, by limiting the axial distance between the fixing member 8 and the axial end portion of the concave opening 52 to be less than or equal to 15% of the axial length of the concave opening 52, the probability of a short anchoring region between the external branch stent 300 and the internal branch stent 6 located near the concave opening 52 due to an excessively long distance from the concave opening 52 can be reduced.

Referring to FIG. 23, in this embodiment, the fixing member 8 is located at an edge of the supporting cover 4. For example, the fixing member 8 may be arranged at the radial edge and/or the axial edge of the supporting cover 4. Since the fixing member 8 is located at the edge of the supporting cover 4, with respect to other regions of the supporting cover 4, the braiding wire 40 located at the edge is less likely to slide relative to the braiding wire 40 in other regions, thereby lowering the risk of damage to the braiding wire 40 caused by mutual friction between the fixing member 8 and other braiding wires 40. Meanwhile, the risk that the mesh openings deform and cannot be fully unfolded because the fixing member 8 hooks other braiding wires 40 can be lowered. Especially when the fixing member 8 is located at the radial edge of the supporting cover 4, even if the concaved stent 100 is subjected to a high radial compression force (for example, the concaved stent 100 is loaded into a deliverer), the fixing member 8 is not easily broken and can always maintain stable connection.

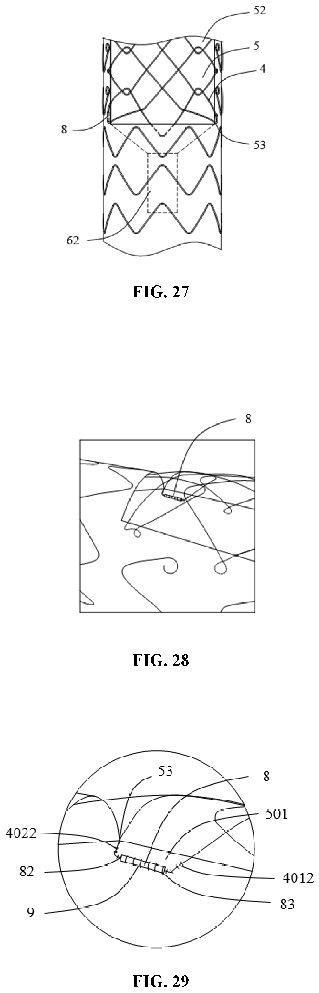

Referring to FIG. 23, and FIG. 27 to FIG. 29, the concave 5 includes an axial edge and a radial edge. The axial edge of the concave 5 is spaced apart and opposite in the axial direction of the concaved stent 100, while the radial edge of the concave 5 is spaced apart and opposite in the radial direction of the concaved stent 100. An edge corner 53 is formed between the axial edge and the radial edge of the concave 5. The fixing member 8 includes a first axial end 82 and a second axial end 83. The first axial end 82 of the fixing member 8 is located at or near the edge corner 53 of the concave 5 (where the first axial end 82 of the fixing member 8 is located near the edge corner 53 of the concave 5, which means that the distance between the first axial end 82 of the fixing member 8 and the edge corner 53 of the concave 5 does not exceed 5 mm). The second axial end 83 of the fixing member 8 is farther away from the edge corner than the first axial end 82, and the fixing member 8 is arranged along the radial edge of the concave 5. It should be noted that the arrangement of the fixing member 8 along the radial edge of the concave 5 does not mean that the fixing member 8 needs to completely fit the radial edge of the concave 5 shown in FIG. 27. A portion of the fixing member 8 is allowed to have a gap from the radial edge of the concave 5. In other embodiments, the fixing member 8 can be arranged along the axial edge of the concave 5. Compared with the arrangement along the axial edge of the concave 5, the fixing member 8 in this embodiment is arranged along the radial edge of the concave 5. When the concaved stent 100 is subjected to a high radial compression force, the fixing member 8 is not easily broken and can always maintain stable connection. In addition, the probability that an end portion of the fixing member 8 damages (such as, punctures) a radial side wall of the concave 5 under radial compression can also be reduced. In other embodiments, the fixing member 8 can be arranged at a position away from the edge corner 53 on the radial edge of the concave 5. Compared with the arrangement of the fixing member 8 at the position away from the edge corner 53, the fixing member 8 in this embodiment is arranged close to the edge corner 53 of the concave 5, so that this can avoid the impact on the lateral bending performance of the concave 5 and help a surgical operator better determine a release position of the concaved stent 100. For example, with the assistance of imaging equipment, by identifying the imaging of the fixing member 8, the position of the axial edge of the concave 5 can be prompted to the surgical operator, which can avoid the obstruction of blood flow in a branch blood vessel 200 due to an inaccurate release position of the concaved stent 100.

Referring to FIG. 23, the supporting cover 4 is connected to the concave 5 through the edge wave angle 421, and the fixing member 8 is arranged on the edge wave angle 421. The wave edge angle 421 is connected to the hooking unit 47. Referring to FIG. 23, the fixing member 8 is arranged on the third wave rod 4212 (referred to as an edge wave rod 4212a) of the edge wave angle 421 directly connected to the radial edge of the concave 5. The edge wave rod 4212a extends substantially along the radial edge of the concave 5 and is connected to the hooking unit 47. For example, the edge wave rod 4212a is connected to the second hook 472 of the hooking unit 47. Due to the relative movement between the first hook 471 and the second hook 472 of the hooking unit 47 in the axial direction, an axial force on or axial movement of other regions of the supporting cover 4 is not easily transmitted to the edge wave angle 421, which can lower the risk of puncturing a blood vessel by the vertex 4211 of the edge wave angle 421.

In addition, the fixing member 8 can be adjacent to or located near the vertex 4211 of the edge wave angle 421 (the fixing member 8 is located near the vertex 4211 of the edge wave angle 421, which means that the distance between the fixing member 8 and the vertex 4211 of the edge wave angle 421 of the concave 5 does not exceed 5 mm). Since the width of the fixing member 8 is greater than the width of each braiding wire 40, a limiting effect can be achieved on the vertex 4211 of the edge wave angle 421, which can further lower the risk of puncturing a blood vessel by the vertex 4211 of the edge wave angle 421.

In this embodiment, the supporting cover 4 includes a first proximal edge wave angle 421c, a second proximal edge wave angle 421d, a first distal edge wave angle 421a, and a second distal edge wave angle 421b. The fixing member 8 in the figure is arranged at the first distal edge wave angle 421a. Since a double-branch-opening stent 61 is arranged near the proximal end of the supporting cover 4 (i.e. near the proximal end of the concave opening 52), and a single-branch-opening stent 62 is arranged near the distal end of the supporting cover 4 (i.e. near the distal end of the concave opening 52), the fixing member 8 is arranged at the distal end of the supporting cover 4 (i.e. the distal end of the concave opening 52) to avoid an increase in the difficulty of sheath withdrawal near the proximal end of the supporting cover 4. In addition, after being implanted, the concaved stent 100 can bend laterally due to compliance with a blood vessel. The radial edge on which the first distal edge wave angle 421a is located is on an outer side of the lateral bend (i.e. a stretched side during bending), and the radial edge on which the second distal edge wave angle 421b is located is on an inner side of the lateral bend (i.e. a compressed side during bending). Therefore, the fixing member 8 arranged at the first distal edge wave angle 421a will not hinder the lateral bending of the supporting cover 4, which can ensure good lateral bending performance of the supporting cover 4. In addition, as shown in the figure, the fixing member 8 is arranged at the first distal edge wave angle 421a. Compared with the fact that the fixing member is arranged at the second distal edge wave angle 421b radially opposite to the first distal edge wave angle 421a, a first side connecting point 46a connected to the first distal edge wave angle 421a can be omitted, so that the mesh opening area of a mesh opening in which the first distal edge wave angle 421a is located is larger than the mesh opening area of a mesh opening in which the second distal edge wave angle 421b is located, which can facilitate the insertion of the guide wire and the external branch stent 300.

Referring to FIG. 27 to FIG. 29, the radial edge of the concave 5 has an inner wall 501. The fixing member 8 is fixedly connected to the inner wall 501 via suturing, bonding, or the like. The advantage of this configuration is that even if the fixing member 8 or a braiding wire 40 in a region adjacent to the fixing member 8 is broken, a side wall of the radial edge of the concave 5 (the side wall is provided with a covering film) can hinder the broken fixing member 8 or the broken braiding wire 40 near the fixing member 8 from puncturing and damaging a tissue wall.

In this embodiment, the fixing member 8 is in sutured connection to the inner wall 501 of the radial edge of the concave 5 through a suture, and the suture forms a plurality of suturing fixing points 9 on an outer surface of the fixing member 8. For example, the suture is wound on the outer surface of the fixing member 8 to form a plurality of suture coils. The plurality of suture coils are arranged in a longitudinal direction of the fixing member 8. Each suture coil fixes the fixing member 8 to the inner wall 501 of the radial edge of the concave 5, thus forming the suturing fixing points 9. By forming the plurality of suturing fixing points 9 on the outer surface of the fixing member 8, it is possible to ensure stable connection between the fixing member 8 and the radial edge of the concave 5. It can be understood that in other embodiments, one or more suturing fixing points 9 may be provided, and threading of the suture may also be different from that illustrated in this embodiment.

Further, referring to FIG. 24 and FIG. 29, in this embodiment, a braiding wire section adjacent to the braiding starting end B0 is recorded as a first section 401. A braiding wire section adjacent to the braiding tail end E0 is recorded as a second section 402. The first section 401 includes a first inner section 4011 located inside the fixing sleeve 81 and a first outer section 4012 connected to the first inner section 4011 and located outside the fixing sleeve 81. The second section 402 includes a second inner section 4021 located inside the fixing sleeve 81 and a second outer section 4022 connected to the second inner section 4021 and located outside the fixing sleeve 81. The first outer section 4012 and/or the second outer section 4022 are fixedly connected to the inner wall 501 of the radial edge of the concave 5. Even if a braiding wire section near an end portion of the fixing sleeve 81 is broken, the broken braiding wire 40 can also be hindered from puncturing and damaging the tissue wall.

Referring to FIG. 25 and FIG. 26, in another embodiment, the fixing member 8 may be arranged in a second mesh region 42. The specific structure of the second mesh region 42 can be found in the description of the first embodiment. The fixing member 8 is arranged on the first direction supporting wire 481 or the second direction supporting wire 482. Whether in a loaded state or an implanted state of the concaved stent 100, the fixing member 8 located on the first direction supporting wire 481 or the second direction supporting wire 482 and the braiding wire section adjacent to the fixing member will not be subjected to an excessive bending force. Therefore, the fixing member 8 and the braiding wire section adjacent to the fixing member are not easily broken, and the supporting cover 4 can be prevented from being damaged or from puncturing and damaging a tissue. For example, the fixing member 8 is located at an intersecting unit 48a (refer to FIG. 25) at the most proximal end in the second mesh region 42 or located at an intersecting unit 48b (refer to FIG. 26) at the most distal end. In other embodiments, the fixing member 8 is located near the intersecting unit 48a at the most proximal end in the second mesh region 42 (the distance from the intersecting unit 48a at the most proximal end does not exceed 5 mm) or located near the intersecting unit 48 at the most distal end (the distance from the intersecting unit 48a at the most distal end does not exceed 5 mm). The fixing member 8 is located here, which can effectively indicate an insertion position of the external branch stent 300. In addition, a positional error can be reserved for the displacement of the concaved stent 100, and the probability of a short anchoring region between the external branch stent 300 and the internal branch stent 6 located near the concave opening 52 due to an excessively long distance from the concave opening 52 can be reduced. Further, when the fixing member 8 is located at the intersecting unit 48 in the second mesh region 42, the fixing member 8 is located on a supporting wire closer to the concave bottom 51 in the intersecting unit 48. In this configuration, after the concaved stent 100 is implanted, another supporting wire is arranged between the fixing member 8 and the tissue wall, so that the fixing member 8 has a movement space, which can reduce stimulation of the fixing member 8 to the tissue wall.

At least part of the outer surface of the fixing member 8 in this embodiment may be provided with a protective structure 70. Especially when the protective structure 70 includes a polymer film layer and completely wraps around the outer surface of the fixing member 8, the fixing member 8 and the braiding wire section adjacent to the fixing member are not easily broken, so that the supporting cover 4 can be prevented from being damaged or from puncturing and damaging a tissue. In addition, even if the fixing member 8 and the braiding wire section adjacent to the fixing member are broken, it is not easy for the supporting cover 4 to directly puncture and damage a tissue.

Referring to FIG. 2, FIG. 23, and FIG. 30, this embodiment further provides a manufacturing method for a concaved stent 100, including:
S10: Provide a main stent 10 and a supporting cover 4.
S20: Connect the supporting cover 4 to the main stent 10.

A manufacturing method for the supporting cover 4 includes:
S11: Provide a braiding wire 40.
S12: Braid the braiding wire 40 into a mesh structure 4a via a plurality of paths, where the mesh structure 4a includes a braiding starting end B0 and a braiding tail end.
S13: Fix the braiding starting end B0 to the braiding tail end E0 of the mesh structure 4a through a fixing member 8.

The mesh structure 4a may be integrally braided from the braiding wire 40, and the mesh structure 4a has only one braiding starting end B0 and one braiding tail end E0. For example, the mesh structure 4a of this embodiment is integrally braided from a single braiding wire 40, and this braiding wire 40 is a single wire. In other embodiments, the mesh structure 4a may be braided from a plurality of braiding wires 40. Each braiding wire 40 can be of a single filament structure or a structure formed by winding a plurality of wires. In other embodiments, the supporting cover 4 may further include a plurality of mesh structures 4a. Each mesh structure 4a may be integrally braided or separately braided and then spliced together.

The plurality of paths include a plurality of first direction paths and a plurality of second direction paths, and step S12 will be explained using the figure as an example.
Step S12 includes:
step A: Braid and form a first direction braided unit along the first direction paths.
step B: Braid and form a second direction braided unit along the second direction paths.
Step A and step B are repeatedly and alternately performed until the mesh structure 4a is formed.

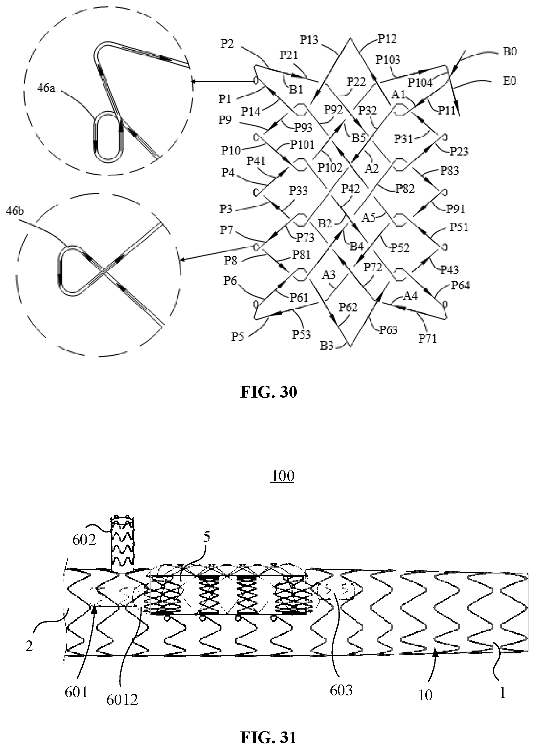

Referring to FIG. 30, the first direction paths refer to paths extending from right to left shown in the figure. For example, P1, P3, P5, P7, and P9 are first direction paths. The second direction paths refer to paths extending from left to right shown in FIG. 30. For example, P2, P4, P6, P8, and P10 are second direction paths. Each of each first direction path and each second direction path may include a plurality of sub-paths. In FIG. 30, P11 to P104 represent a plurality of sub-paths. For example, P1 sequentially includes sub-paths P11, P12, P13, and P14; P2 sequentially includes sub-paths P21, P22, and P23; P3 sequentially includes sub-paths P31, P32, and P33; P4 sequentially includes sub-paths P41, P42, and P43; P5 sequentially includes sub-paths P51, P52, and P53; P6 sequentially includes sub-paths P61, P62, P63, and P64; P7 sequentially includes sub-paths P71, P72, and P73; P8 sequentially includes sub-paths P81, P82, and P83; P9 sequentially includes sub-paths P91, P92, and P93; and P10 sequentially includes sub-paths P101, P102, P103, and P104.

For example, starting from the braiding starting end B0, braiding is performed sequentially along the sub-paths P11, P12, P13, and P14 of the path P1 to form a first direction braided unit A1; braiding is performed sequentially along the sub-paths P21, P22, and P23 of the path P2 to form a second direction braided unit B1; braiding is performed sequentially along the sub-paths P31, P32, and P33 of the path P3 to form a first direction braided unit A2; braiding is performed sequentially along the sub-paths P41, P42, and P43 of the path P4 to form a second direction braided unit B2; braiding is performed sequentially along the sub-paths P51, P52, and P53 of the path P5 to form a first direction braided unit A3; braiding is performed sequentially along the sub-paths P61, P62, P63, and P64 of the path P6 to form a second direction braided unit B3; braiding is performed sequentially along the sub-paths P71, P72, and P73 of the path P7 to form a first direction braided unit A4; braiding is performed sequentially along the sub-paths P81, P82, and P83 of the path P8 to form a second direction braided unit B4; braiding is performed sequentially along the sub-paths P91, P92, and P93 of the path P9 to form a first direction braided unit A5; and braiding is performed sequentially along the sub-paths P101, P102, P103, and P104 of the path P10 to form a second direction braided unit B5. The braiding ends at the braiding tail end B0. Finally, the braiding starting end B0 and the braiding tail end E0 are cut to suitable lengths and are then fixed to each other through the fixing member 8.

The first direction braided units and the second direction braided units are alternately formed and connected to each other, and at least one intersection point is formed between a first direction braided unit and a second direction braided unit that are adjacent to each other.

It can be understood that in other embodiments, the first direction paths may be from left to right, and the second direction paths may be from right to left. In other embodiments, the braiding starting end and the braiding tail end may be completely different from those in this embodiment. For example, E0 can serve as the braiding starting end, and B0 can serve as the braiding tail end, for braiding in a direction opposite to the arrow directions in FIG. 30. The mesh structure 4a can also be manufactured. For another example, a braiding starting end and a braiding tail end can be selected near an intersection between the sub-path P22 and the sub-path P102, or any other suitable braiding starting end and braiding tail end can be used, as long as they can be braided to form the mesh structure 4a.

A side connecting member 46 can also be arranged between the first direction braided unit and the second direction braided unit that are adjacent to each other. For example, a second side connecting member 46b is arranged between the first direction braided unit A1 and the second direction braided unit B1 that are located at an axial end portion; a second side connecting member 46b is arranged between the first direction braided unit A3 and the second direction braided unit B3 that are located at another axial end portion; a second side connecting member 46b is arranged between the first direction braided unit A4 and the second direction braided unit B3. Except for the braiding starting end B0 and the braiding tail end, first side connecting members 46a are arranged between other adjacent first direction braided units and second direction braided units. In this embodiment, the shape of the first side connecting member 46a is different from the shape of the second side connecting member 46b. Referring to FIG. 30, the first side connecting member 46a is substantially triangular, while the second side connecting member 46b is substantially elliptical. In other embodiments, they can be in any other suitable shapes, specifically as described in the first embodiment. In other embodiments, they can be in the same shape.

It can be understood that the structures of the concaved stent 100 in the first embodiment to the fifth embodiment may be different from the structure exemplified above. Referring to FIG. 31, in some embodiments, the concaved stent 100 includes a first branch stent 601 and a second branch stent 602. The first branch stent 601 is disposed within the main stent 10 and is communicated with the concave 5. One end of the second branch stent 602 is fixedly connected and communicated with the main stent 10, and another end is a free end having a branch opening. The free end of the second branch stent 602 is disposed outside the main stent 10 and is configured to be implanted into a branch blood vessel 200 or connected to another stent. The longitudinal extension direction of the second branch stent 602 is intersected with the axial direction of the concaved stent 100. The second branch stent 602 and the first branch stent 601 are both communicated with the inner cavity of the main stent 10. After the second branch stent 602 is communicated with a corresponding branch blood vessel 200, the concave 5 of the concaved stent 100 is aligned with an opening of another branch blood vessel 200, which can mitigate the situation where it is difficult to align the concave 5 with the corresponding branch blood vessel 200 due to the deflection of the concaved stent 100 after release. In addition, the free end of the second branch stent 602 is disposed outside the main stent 10, so that the second branch stent 602 can be implanted into the branch blood vessel 200 without occupying the space of the internal cavity of the main stent 10, thereby reducing the occupation of the internal cavity of the main stent 10 by the branch stent and increasing the blood flow of the blood in the internal cavity of the main stent 10. Compared with the radial side-by-side arrangement of the first branch stent 601 and the second branch stent 602 inside the main stent 10, this embodiment configures the free end of the second branch stent 602 outside the main stent 10, which can also enable the guide wire or the external branch stent 300 to enter the corresponding branch stent more accurately, so as to lower the risk that the guide wire accidentally enters another branch stent. For example, the longitudinal extension direction of the second branch stent 602 is perpendicular to the axial direction.

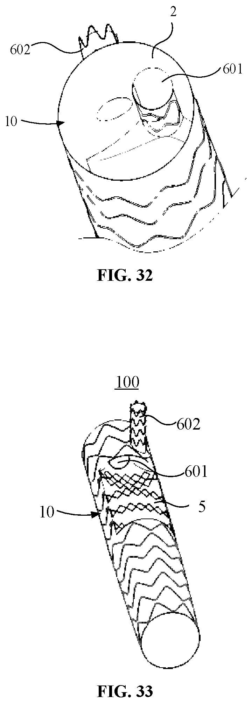

Referring to FIG. 31 and FIG. 32, in some embodiments, the first branch stent 601 is arranged inside the main stent 10 and is communicated with the concave 5. The second branch stent 602 and the first branch stent 601 are arranged on one axial side of the concave 5. For example, the second branch stent 602 and the first branch stent 601 are arranged at the proximal end of the concave 5. That is, both the second branch stent 602 and the first branch stent 601 are arranged at the proximal section 2 of the main stent 10. If the first branch stent 601 and the second branch stent 602 are arranged side by side in the radial direction at the proximal end of the concave 5, a portion of the first branch stent 601 adjacent to the second branch stent 602, a portion of the second branch stent 602 adjacent to the first branch stent 601, and the inner wall of the main stent 10 enclose a triangular region. This triangular region can be impacted by the blood flow and form a vortex, which will affect the flowing direction of the blood in the inner cavity of the main stent 10. In this embodiment, the second branch stent 602 is arranged outside the main stent 10, and the first branch stent 601 and the second branch stent 602 on the axial side of the concave 5 will not form a triangular region with the main stent 10, thus reducing the impact on the flowing direction of the blood in the inner cavity of the main stent 10.

Referring to FIG. 31, in some embodiments, a guide section 6012 is formed at a distal end of the first branch stent 601, and the cross-sectional area of the guide section 6012 extends from the proximal end to the distal end in a gradually enlarged manner. The distal end of the first branch stent 601 is designed as a horn-shaped guide section 6012. The guide section 6012 can play a role in guiding the entry of the guide wire or the external branch stent 300.

Referring to FIG. 33, for example, the proximal end of the concaved stent 100 is an upper part, and the distal end of the concaved stent 100 is a lower part. The second branch stent 602 is arranged on a right side of the first branch stent 601 to better adapt to three branch blood vessels 200 near the aortic arch 300. The second branch stent 602 is configured to be implanted into the leftmost branch blood vessel 200 in FIG. 1. After the second branch stent 602 is implanted into the corresponding branch blood vessel 200, a positioning effect can be achieved on the concaved stent 100 to an extent, so that the concave 5 is aligned with the other two branch blood vessels 200. After the second branch stent 602 is implanted into the corresponding branch blood vessel 200, the second branch stent 602 will neither interfere with implantation of corresponding external branch stents 300 into the other two branch blood vessels 200, nor interfere with connection between the external branch stents 300 and corresponding internal branch stents 6.

In some embodiments, the first branch stent 601 can also be arranged outside the main stent 10. For example, one end of the first branch stent 601 is fixedly connected and communicated with the main stent 10, and another end is a free end having a branch opening. The free end of the first branch stent 601 is arranged outside the main stent 10, and the longitudinal extension direction of the first branch stent 601 is intersected with the axial direction. The first branch stent 601 and the second branch stent 602 are arranged in the axial direction of the concaved stent 100 and are disposed at the proximal section 2. That is, the first branch stent 601 and the second branch stent 602 are respectively configured to be communicated with two branch blood vessels 200. After the first branch stent 601 and the second branch stent 602 are communicated with the corresponding branch blood vessels 200, the concave 5 of the concaved stent 100 is aligned with the opening of the other branch blood vessel 200, which can mitigate the situation where it is difficult to align the concave 5 with the corresponding branch blood vessel 200 due to the deflection of the concaved stent 100 after release. In addition, the branch openings of the free ends of the first branch stent 601 and the second branch stent 602 are disposed outside the main stent 10, so that the first branch stent 601 and the second branch stent 602 can be implanted into the branch blood vessels 200 without occupying the space of the internal cavity of the main stent 10, thereby further reducing the occupation of the internal cavity of the main stent 10 by the branch stents and increasing the blood flow of the blood in the internal cavity of the main stent 10. In other embodiments, the concaved stent 100 further includes a third branch stent 83. The third branch stent 83 is arranged at the distal end of the concave 5. The third branch stent 83 can be arranged outside the main stent 10, like the second branch stent 602. Or, the third branch stent 83 is disposed within the inner cavity of the main stent 10.

In other embodiments, the first branch stent 601 and the second branch stent 602 may be arranged at the distal end of the concave 5, and the third branch stent 83 may be arranged at the proximal end of the concave 5.

The above specific embodiments are only some embodiments of the present invention and do not limit the present invention. This specification cannot exhaustively list all the embodiments of the concept of the present invention, and some features of different embodiments described above may be replaced or combined with each other. Those skilled in the art can also make simple substitutions according to actual needs. The protection scope required by the concept of the present invention shall prevail.

## Claims

1. A concaved stent, comprising:
a main stent, wherein the main stent is tubular and has an inner cavity, a side surface of the main stent is recessed toward the inner cavity to form a concave, and the concave comprises a concave bottom; and
a supporting cover connected to the main stent, wherein a radial gap in a radial direction of the concaved stent is formed between at least a portion of the supporting cover and the concave bottom, the supporting cover comprises a mesh structure braided from a braiding wire, the mesh structure comprises a plurality of deformable mesh openings, and the concave communicates with the outside via the mesh openings; the mesh structure comprises a braiding starting end and a braiding tail end, and the braiding starting end and the braiding tail end are fixed to each other through a fixing member.

2. The concaved stent according to claim 1, wherein the fixing member is located at a radial edge and/or an axial edge of the supporting cover.

3. The concaved stent according to claim 1, wherein the concave comprises an axial edge and a radial edge; an edge corner is formed between the axial edge and the radial edge of the concave; the fixing member comprises a first axial end and a second axial end; the first axial end of the fixing member is located at the edge corner of the concave or close to the edge corner of the concave; the second axial end of the fixing member is farther away from the edge corner than the first axial end; and the fixing member is arranged along the radial edge of the concave.

4. The concaved stent according to any one of claims 1 to 3, wherein the supporting cover comprises an edge wave angle located at an axial end portion; the edge wave angle comprises a vertex; the vertex of the edge wave angle is connected to the edge corner of the concave; the fixing member is arranged on the edge wave angle; and the fixing member is adjacent or close to the vertex of the edge wave angle.

5. The concaved stent according to claim 4, wherein the edge wave angle is connected to a hooking unit; the hooking unit comprises a first hooking member and a second hooking member that are movable relative to each other in an axial direction.

6. The concaved stent according to any one of claims 1 to 4, wherein the concave comprises a radial edge; the radial edge of the concave has an inner wall; and the fixing member is fixedly connected to the inner wall.

7. The concaved stent according to claim 6, wherein the fixing member is in sutured connection to the inner wall through a suture; and the suture forms a plurality of suturing fixing points on an outer surface of the fixing member.

8. The concaved stent according to claim 6, wherein the fixing member comprises a fixing sleeve comprising a sleeve inner cavity; the braiding starting end and the braiding tail end are fixed in the sleeve inner cavity; a braiding wire section adjacent to the braiding starting end is recorded as a first section; a braiding wire section adjacent to the braiding tail end is recorded as a second section; the first section comprises a first inner section located inside the fixing sleeve and a first outer section connected to the first inner section and located outside the fixing sleeve; the second section comprises a second inner section located inside the fixing sleeve and a second outer section connected to the second inner section and located outside the fixing sleeve; and the first outer section and/or the second outer section are fixedly connected to the inner wall.

9. The concaved stent according to any one of claims 1 to 8, wherein in a circumferential direction of the concaved stent, the supporting cover comprises a second mesh region and at least two first mesh regions respectively connected to two sides of the second mesh region; the second mesh region comprises a plurality of first direction supporting wires arranged at intervals and a plurality of second direction supporting wires arranged at intervals; the first direction supporting wires and the second direction supporting wires overlap each other to form a plurality of columns of intersecting units and a plurality of columns of deformable mesh openings; and at least one fixing member is disposed in the second mesh region.

10. The concaved stent according to any one of claims 1 to 9, wherein the concave comprises a concave opening; the axial distance between at least one fixing member and the proximal end of the concave opening is less than the axial distance between the fixing member and the distal end of the concave opening; or, the axial distance between at least one fixing member and the proximal end of the concave opening is greater than the axial distance between the fixing member and the distal end of the concave opening.

11. The concaved stent according to claim 10, wherein when the axial distance between the fixing member and the proximal end of the concave opening is less than the axial distance between the fixing member and the distal end of the concave opening, the ratio of the axial distance between the fixing member and the proximal end of the concave opening to the axial length of the concave opening ranges from 5% to 15%; and when the axial distance between the fixing member and the proximal end of the concave opening is greater than the axial distance between the fixing member and the distal end of the concave opening, the ratio of the axial distance between the fixing member and the distal end of the concave opening to the axial length of the concave opening ranges from 5% to 15%.

12. The concaved stent according to any one of claims 1 to 9, wherein the braiding starting end and the braiding tail end are fixed within the fixing member; the braiding starting end and the braiding tail end are opposite to each other in a longituginal direction of the braiding wire; and the braiding starting end and the braiding tail end are abutted with each other or spaced apart from each other.

13. The concaved stent according to any one of claims 1 to 9, further comprising one or more branch stents connected to the main stent; and the branch stents are disposed within the main stent, or at least part of the branch stents is disposed outside the main stent.

14. A manufacturing method for the concaved stent according to any one of claims 1 to 13, comprising:
providing a main stent and a supporting cover; and
connecting the supporting cover to the main stent,
wherein a manufacturing method for the supporting cover comprises:
providing a braiding wire;
braiding the braiding wire into a mesh structure via a plurality of paths, wherein the mesh structure comprises a braiding starting end and a braiding tail end; and
fixing the braiding starting end to the braiding tail end of the braiding wire through a fixing member.

15. The manufacturing method for the concaved stent according to claim 14, wherein the mesh structure is integrally braided from the braiding wire, and the mesh structure has only one braiding starting end and one braiding tail end.

16. The manufacturing method for the concaved stent according to claim 14, wherein the plurality of paths comprise a plurality of first direction paths and a plurality of second direction paths, and the braiding the braiding wire into a mesh structure via a plurality of paths comprises:
step A: braiding and forming a first direction braided unit along the first direction paths; and
step B: braiding and forming a second direction braided unit along the second direction paths, and
repeatedly and alternately performing step A and step B until the mesh structure is formed, wherein the first direction braided unit and the second direction braided unit are alternately formed and connected to each other, and at least one intersection point is formed between the first direction braided unit and the second direction braided unit that are adjacent to each other.

17. The concaved stent according to any one of claims 1 to 13, wherein a protective structure is arranged on a surface of part or all of the supporting cover.

18. The concaved stent according to claim 17, wherein the protective structure comprises one or more protective layers among a metal layer, a polymer layer, a ceramic layer, and a composite material layer that are arranged on a surface of the supporting cover; and the composite material layer is made of one or more materials among metal, polymer, and ceramic.

19. The concaved stent according to claim 17, wherein the mesh structure comprises a plurality of overlapping points formed by overlapping of the braiding wire; and at at least some of the overlapping points, a protective structure is arranged on a surface of the braiding wire; and/or, the mesh openings are deformable mesh openings; and the protective structure is arranged on inner walls of at least some of the mesh openings.

20. The concaved stent according to claim 17, wherein the mesh structure comprises a mesh main body and a side connecting member arranged on a radial side edge of the mesh main body; a protective structure is arranged on a surface of the mesh main body; and no protective structure is arranged on at least a portion of the side connecting member.

21. The concaved stent according to any one of claims 17 to 20, wherein the protective structure comprises a protective unit; the supporting cover comprises at least one column of hooking units; each hooking unit comprises a first hook and a second hook that are sequentially hooked to each other in a direction from a proximal end to a distal end; a hooking gap is formed between a valley of the first hook and a peak of the second hook, so that the valley of the first hook and the peak of the second hook are movable relative to each other in the axial direction; and at least one hooking unit is provided with the protective unit.

22. The concaved stent according to claim 21, wherein the protective structure comprises a first protective unit and/or a second protective unit; the first protective unit is arranged on one side facing the hooking gap of the valley of the first hook; and the second protective unit is arranged on one side facing the hooking gap of the peak of the second hook.

23. The concaved stent according to claim 21, wherein a first protective unit is arranged on one side facing the hooking gap of the valley of the first hook; a second protective unit is arranged on one side facing the hooking gap of the peak of the second hook; the first protective unit and the second protective unit jointly shield part of the hooking gap, and a through hole is formed between the first protective unit and the second protective unit; the valley of the first hook and the peak of the second hook are configured to move away from each other in the axial direction to drive the through hole to be enlarged for insertion of an external branch stent; and in a naturally expanded state, the area of the through hole is smaller than the cross-sectional area of the external branch stent.

24. The concaved stent according to any one of claims 17 to 23, wherein the protective structure comprises a protective unit; and the protective unit comprises one or more of a sheet-like structure, a strip-shaped structure, and a block-shaped structure.

25. The concaved stent according to claim 22, wherein at least one of the first protective unit and the second protective unit comprises a main sheet and an extension sheet; both the main sheet and the extension sheet are sheet-like structures; one end of the main sheet is connected to the hooking unit, and another end extends into the hooking gap and is connected to the extension sheet; the extension sheet has a free end and extends toward the concave bottom; and the free end of the extension sheet is located between the supporting cover and the concave bottom.

26. The concaved stent according to claim 22, wherein at least one of the first protective unit and the second protective unit comprises a sheet-like structure, and the sheet-like structure has a movable end which comprises a thin-walled region; the thickness of the thin-walled region is less than the thickness of another region of the protective unit; and when an external branch stent is inserted into the hooking gap from the outside toward the concave, at least a portion of the thin-walled region deforms relative to another region of the sheet-like structure and extends toward the concave bottom.

27. The concaved stent according to claim 26, wherein the thin-walled region comprises at least one crack-prone strip; the crack-prone strip cracks to form a crack when the external branch stent is inserted into the hooking gap from the outside toward the concave bottom; and at least a portion adjacent to the crack of the thin-walled region deforms relative to another region of the sheet-like structure and extends toward the concave bottom.

28. The concaved stent according to claim 21, wherein the protective structure comprises a first protective unit and a second protective unit; the first protective unit and the second protective unit jointly shield the hooking gap; or, the protective structure comprises a third protective unit which comprises a polymer covering film; the polymer covering film completely covers one or more hooking gaps; the valley of the first hooking unit and/or the peak of the second hooking unit are movable in the axial direction; and an insertion hole is formed in at least a portion of the supporting cover.

29. A concaved stent, comprising:
a main stent, wherein the main stent is tubular and has an inner cavity, a side surface of the main stent is recessed toward the inner cavity to form a concave, and the concave comprises a concave bottom; and
a supporting cover connected to the main stent, wherein a radial gap in a radial direction of the concaved stent is formed between at least a portion of the supporting cover and the concave bottom; the supporting cover is configured to communicate the concave with the outside; and a protective structure is arranged on a surface of part or all of the supporting cover.

30. The concaved stent according to claim 29, wherein the protective structure comprises one or more protective layers among a metal layer, a polymer layer, a ceramic layer, and a composite material layer that are arranged on a surface of the supporting cover; and the composite material layer is made of one or more materials among metal, polymer, and ceramic.

31. The concaved stent according to claim 29, wherein the supporting cover comprises a mesh structure braided from a braiding wire; the mesh structure comprises a plurality of mesh openings and comprises a plurality of overlapping points formed by overlapping of the braiding wire; and at at least some of the overlapping points, a protective structure is arranged on a surface of the braiding wire; and/or, the mesh openings are deformable mesh openings; and the protective structure is arranged on inner walls of at least some of the mesh openings.

32. The concaved stent according to claim 29, wherein the supporting cover comprises a mesh structure; the mesh structure comprises a mesh main body and a side connecting member arranged on a radial side edge of the mesh main body; a protective structure is arranged on a surface of the mesh main body; and no protective structure is arranged on at least a portion of the side connecting member.

33. The concaved stent according to any one of claims 29 to 32, wherein the protective structure comprises a protective unit; the supporting cover comprises at least one column of hooking units; each hooking unit comprises a first hook and a second hook that are sequentially hooked to each other in a direction from a proximal end to a distal end; a hooking gap is formed between a valley of the first hook and a peak of the second hook, so that the valley of the first hook and the peak of the second hook are movable relative to each other in the axial direction; and at least one hooking unit is provided with the protective unit.

34. The concaved stent according to claim 33, wherein the protective structure comprises a first protective unit and/or a second protective unit; the first protective unit is arranged on one side facing the hooking gap of the valley of the first hook; and the second protective unit is arranged on one side facing the hooking gap of the peak of the second hook.

35. The concaved stent according to claim 33, wherein a first protective unit is arranged on one side facing the hooking gap of the valley of the first hook; a second protective unit is arranged on one side facing the hooking gap of the peak of the second hook; the first protective unit and the second protective unit jointly shield part of the hooking gap, and a through hole is formed between the first protective unit and the second protective unit; the valley of the first hook and the peak of the second hook are configured to move away from each other in the axial direction to drive the through hole to be enlarged for insertion of an external branch stent; and in a naturally expanded state, the area of the through hole is smaller than the cross-sectional area of the external branch stent.

36. The concaved stent according to any one of claims 29 to 35, wherein the protective structure comprises a protective unit; and the protective unit comprises one or more of a sheet-like structure, a strip-shaped structure, and a block-shaped structure.

37. The concaved stent according to claim 34, wherein at least one of the first protective unit and the second protective unit comprises a main sheet and an extension sheet; both the main sheet and the extension sheet are sheet-like structures; one end of the main sheet is connected to the hooking unit, and another end extends into the hooking gap and is connected to the extension sheet; the extension sheet has a free end and extends toward the concave bottom; and the free end of the extension sheet is located between the supporting cover and the concave bottom.

38. The concaved stent according to claim 34, wherein at least one of the first protective unit and the second protective unit comprises a sheet-like structure, and the sheet-like structure has a movable end which comprises a thin-walled region; the thickness of the thin-walled region is less than the thickness of another region of the protective unit; and when an external branch stent is inserted into the hooking gap from the outside toward the concave, at least a portion of the thin-walled region deforms relative to another region of the sheet-like structure and extends toward the concave bottom.

39. The concaved stent according to claim 38, wherein the thin-walled region comprises at least one crack-prone strip; the crack-prone strip cracks to form a crack when the external branch stent is inserted into the hooking gap from the outside toward the concave bottom; and at least a portion, adjacent to the crack, of the thin-walled region deforms relative to another region of the sheet-like structure and extends toward the concave bottom.

40. The concaved stent according to claim 33, wherein the protective structure comprises a first protective unit and a second protective unit; the first protective unit and the second protective unit jointly shield the hooking gap; or, the protective structure comprises a third protective unit which comprises a polymer covering film; the polymer covering film completely covers one or more hooking gaps; the valley of the first hooking unit and/or the peak of the second hooking unit are movable in the axial direction; and an insertion hole is formed in at least a portion of the supporting cover.
